# EUROPEAN PATENT SPECIFICATION

(11) **EP 2 182 943 B1**
(45) Date of publication and mention of the grant of the patent: **26.10.2016**
(21) Application number: 08782121.1
(22) Date of filing: 21.07.2008
(51) Int. Cl.: A61K 31/40, A61K 38/00

(54) **METHODS AND COMPOSITIONS FOR TREATING FIBROSIS RELATED DISORDERS USING IL-17 ANTAGONISTS**
VERFAHREN UND ZUSAMMENSETZUNGEN ZUR BEHANDLUNG VON FIBROSEBEDINGTEN ERKRANKUNGEN MIT IL-17-ANTAGONISTEN
PROCÉDÉS ET COMPOSITIONS POUR TRAITER DES TROUBLES ASSOCIÉS À UNE FIBROSE EN UTILISANT DES ANTAGONISTES DE L'IL-17

(30) Priority: 23.07.2007 US 951270 P; 13.08.2007 US 955414 P
(43) Date of publication of application: 12.05.2010
(73) Proprietor: Janssen Biotech, Inc., Horsham, PA 19044 (US)
(72) Inventor: DUDAS, Paul, Malvern, Pennsylvania 19355 (US); SAGUE, Sarah, Malvern, PA 19355 (US); ELLOSO, M. Merle, Malvern, Pennsylvania 19355 (US); FARRELL, Francis, Radnor,PA 19087 (US)
(74) Representative: Goodfellow, Hugh Robin
(86) International application number: PCT/US2008/070588
(87) International publication number: WO 2009/015063

(56) References cited:
- WO-A1-2006/013107
- WO-A2-2006/088833
- US-A1- 2006 106 038
- US-A1- 2006 257 403
- US-A1- 2007 048 257
- US-A1- 2007 160 576
- LAAN ET AL.: 'IL-17-induced cytokine release in human bronchial epithelial cells in vitro:role of mitogen-activated protein (MAP) kinases' BR.J.PHARM. vol. 133, 2001, pages 200 - 206, XP008129916

## Description

### Field of the Invention

The present invention relates methods and compositions for treating interleukin-17 (IL-17) related conditions in patients having fibrosis using IL-17 antagonists, such as small molecule IL-17 antagonists or protein IL-17 antagonists, such as antibodies, including specified portions or variants, specific for at least one IL-17 protein, mutein or fragment thereof, including therapeutic formulations, administration and devices.

### RELATED ART

Renal fibrosis can develop from either an acute insult (ex. graft ischemia/reperfusion) (Freese et al., Nephrol Dial Transplant 2001; 16: 2401-2406) or chronic condition (ex. diabetes)(Ritz et al., Nephrol Dial Transplant 1996; 11 Suppl 9: 38-44). The pathogenesis is typically characterized by an initial inflammatory response followed by sustained fibrogenesis of the glomerular filtration apparatus and tubular interstitium (Liu Y, Kidney Int 2006; 69: 213-217). Tubulointerstitial fibrosis has been shown to play a critical role in the pathogenesis of renal injury to end-stage renal failure and the proximal tubule cell has been revealed as a central mediator (Phillips and Steadman, Histol Histopathol 2002; 17: 247-52)(Phillips, Chang Gung Med J 2007; 30(1): 2-6). Fibrogenesis in the tubulointerstitial compartment is mediated in part by activation of resident fibroblasts, which secrete pro-inflammatory cytokines that stimulate the proximal tubule epithelium to secrete local inflammatory and fibrogenic mediators.

Additionally, chemotactic cytokines are secreted by fibroblasts and epithelial cells and provide a directional gradient guiding the infiltration of monocytes/macrophages and T-cells into the tubulointerstitium. The inflammatory infiltrate produces additional fibrogenic and inflammatory cytokines that further activate fibroblast and epithelial cytokine release while also stimulating the epithelium to undergo a phenotypic transition in which the cells deposit excess extracellular matrix components (Simonson MS, Kidney Int 2007; 71: 846-854).

Interleukin-17 (IL-17) is a T cell-derived pro-inflammatory cytokine that is upregulated during human renal allograft rejection (Van Kooten et al., J Am Soc Nephrol 1998; 9: 1526-1534)(Loong et al., J Path 2002; 197: 322-332). IL-17 stimulates the production of the pro-inflammatory mediators IL-6, IL-8, complement component C3, IL-17 and RANTES by proximal tubular epithelium (Van Kooten et al., J Am Soc Nephrol 1998; 9: 1526-1534)(Woltman et al., J Am Nephrol 2000; 11: 2044-55). These factors, in turn, mediate the recruitment of other inflammatory cell-types into the interstitium that contribute to the maintenance of the inflammatory/immune response and, if not suppressed, the onset of fibrosis and chronic allograft nephropathy (Racusen et al., Kidney Int 1999; 55: 713-23)(Mannon, Am J Transpl 2006; 6: 867-75). It is however unclear if IL-17 only indirectly impacts epithelial cell function by inducing the secretion of soluble mediators that possess detrimental autocrine or paracrine activity or if IL-17 has a direct receptor-mediated pro-fibrotic effect on the proximal tubule epithelium.

Accordingly, there is a need to provide human antibodies specific for human IL-17 for use in therapy to diminish or eliminate symptoms of IL-17 related fibrosis, as well as improvements over known antibodies or fragments thereof.

### SUMMARY OF THE INVENTION

Here disclosed are methods and compositions for treating IL-17 related conditions in patients having fibrosis using IL-17 antagonists, such as small molecule IL-17 antagonists or protein IL-17 antagonists, such as antibodies, including specified portions or variants, specific for at least one human interleukin-17 (IL-17) protein, such as IL-17A, IL-17F, or muteins thereof (e.g., but not limited to, one of SEQ ID NOS: 1-4) or fragment thereof, including therapeutic formulations, administration and devices.

A method of the present invention provides for the inhibiting at least interleukin-17 (IL-17) in a patient having kidney or renal fibrosis using at least one IL-17 antagonist, comprising administering an IL-17 inhibiting effective amount of at least one IL-17 antagonists. The IL-17 antagonist is an antibody.

This invention discloses IL-17 as a pro-fibrotic factor capable of acting directly on the renal proximal tubule epithelium to induce transition to a pro-fibrotic phenotype. The ability of IL-17 to mediate renal fibrogenesis indirectly through IL-17-induced secretion of pro-fibrotic soluble mediators is also demonstrated. IL-17 stimulated the expression of pro-fibrotic genes CTGF, CD44 and TGFβR1 and, in parallel, decreased the expression of the pro-epithelial marker e-cadherin. A loss of epithelial phenotype as indicated by downregulation of e-cadherin has previously been demonstrated as a hallmark event in the onset of renal fibrosis while CTGF, CD44 and TGFβR1 have been shown to play active roles in the pathogenesis (Thomas et al., Adv Chr Kid Dis 2005; 12(2): 177-86)(Eitner and Floege Curr Op Invest Drugs 2005; 6(3): 255-61)(Florquin and Rouschop Kid Int Suppl 2003 Oct; (86): S15-20). Additionally, IL-17 stimulated the secretion of pro-inflammatory cytokines (IL-6 and IL-8) and several factors that function as immune cell chemoattractants and mediators of immune cell interaction with the resident epithelium (fractalkine, GM-CSF and G-CSF)(Stievano L et al., Crit Rev Immunol 2004; 24(3): 205-28)(Gasson JC et al., Prog Clin Biol Res 1990; 352: 375-84)(Eyles JL et al., Nat Clin Pract Rheumatol 2006 Sep; 2(9): 500-10). A component of renal fibrogenesis and a central contributor to diminished renal function is the disruption of normal epithelial architecture (Thomas MC et al., Adv Chron Kid Dis 2005; 12(2): 177-86). IL-17 substantially disrupted cell-cell junctions and diminished epithelial integrity in confluent epithelial monolayers as indicated by a reduction in the tight junctional protein zonula occludens-1 (ZO-1). Preliminary data suggest that the aforementioned IL-17-induced secretion of pro-inflammatory/fibrotic mediators and ZO-1 downregulation can be suppressed following co-administration with a neutralizing antibody specific for human IL-17. We therefore propose that neutralization of IL-17 activity may be beneficial for the treatment of renal pathologies manifesting an inflammatory and/or fibrotic component.

In such a method, the antibody can comprise at least one variable region comprising at least one heavy chain variable region and at least one light chain, said IL-17 antibody comprising both heavy chain and light chain variable regions that bind at least one of SEQ ID NOS: 1-3. In such a method the antibody binds IL-17 with an affinity of at least one selected from at least 10⁻⁹ M, at least 10⁻¹⁰ M, at least 10⁻¹¹ M, or at least 10⁻¹² M. The antibody can substantially modulate at least one activity of at least one IL-17 polypeptide. The small molecule or protein can be provided as a composition further comprising at least one pharmaceutically acceptable carrier or diluent.

The method can further comprise administering at least one compound or polypeptide selected from at least one of a detectable label or reporter, a TNF antagonist, an anti-infective drug, a cardiovascular (CV) system drug, a central nervous system (CNS) drug, an autonomic nervous system (ANS) drug, a respiratory tract drug, a gastrointestinal (GI) tract drug, a hormonal drug, a drug for fluid or electrolyte balance, a hematologic drug, an antineoplastic, an immunomodulatory drug, an opthalmic, otic or nasal drug, a topical drug, a nutritional product, a cytokine, or a cytokine antagonist.

In such a method, the inhibiting effective amount can be 0.001-50 mg/kilogram of said cells, tissue, organ or animal, such as but not limited to 0.01-20 mg/kg, 1-10, 1-3, 1-5, .1-1, .2-2 mg/kg and the like.

In such a method, the administering can be by at least one mode selected from parenteral, subcutaneous, intramuscular, intravenous, intraarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal.

In such a method, the fibrosis can be organ specific fibrosis or systemic fibrosis. The organ specific fibrosis can be associated with at least one of lung fibrosis, liver fibrosis, kidney fibrosis (or renal fibrosis), heart fibrosis, vascular fibrosis, skin fibrosis, eye fibrosis, bone marrow fibrosis or other fibrosis. The lung fibrosis can be associated with at least one of idiopathic pulmonary fibrosis, drug induced pulmonary fibrosis, asthma, sarcoidosis or chronic obstructive pulmonary disease. The liver fibrosis can be associated with at least one of cirrhosis, schistomasomiasis or cholangitis. The cirrhosis can be selected from alcoholic cirrhosis, post-hepatitis C cirrhosis, primary biliary cirrhosis. The cholangitis is sclerosing cholangitis. The kidney fibrosis can be associated with at least one of diabetic nephropathy, IgA nephropathy, transplant nephropathy, or lupus nephritis. The heart fibrosis can be associated with at least one type of myocardial infarction. The vascular fibrosis can be associated with at least one of postangioplasty arterial restenosis, or atherosclerosis. The skin fibrosis can be associated with at least one of burn scarring, hypertrophic scarring, keloid, or nephrogenic fibrosing dermatopathy. The eye fibrosis can be associated with at least one of retro-orbital fibrosis, postcataract surgery or proliferative vitreoretinopathy. The bone marrow fibrosis can be associated with at least one of idiopathic myelofibrosis or drug induced myelofibrosis. The other fibrosis can be selected from Peyronie's disease, Dupuytren's contracture or dermatomyositis. The systemic fibrosis can be selected from systemic sclerosis and graft versus host disease.

The present invention provides isolated human, primate, rodent, mammalian, chimeric, humanized and/or CDR-grafted anti-IL-17 antibodies and other immunoglobulin derived proteins, fragments, cleavage products and other specified portions and variants thereof, as well as anti-IL-17 antibody compositions, encoding or complementary nucleic acids, vectors, host cells, compositions, formulations, devices, transgenic animals, transgenic plants, and methods of making and using thereof, as described and enabled herein, in combination with what is known in the art. In addition to the composition of the antibodies of the invention as described herein, the antibody of the present invention is defined by its affinity for human IL-17, specificity for human IL-17 and ability to block bioactivity of human IL-17.

The present invention also provides at least one isolated anti-IL-17 antibody, such as, but not limited to at least one an antibody, antibody fusion protein or fragment, as described herein. An antibody according to the present invention includes any protein or peptide containing molecule that comprises at least a portion of an immunoglobulin molecule, such as but not limited to, at least one ligand binding domain, such as but not limited to, a heavy chain or light chain variable region, a complementarity determining region (CDR) of a heavy or light chain or a ligand binding portion thereof, further optionally comprising at least CH1, hinge, CH2, or CH3 of an human immunoglobulin. The at least one antibody amino acid sequence can further optionally comprise at least one specified substitution, insertion or deletion as described herein or as known in the art.

Also disclosed herein are isolated nucleic acid molecules comprising, complementary, or hybridizing to, a polynucleotide encoding specific anti-IL-17 antibodies, comprising at least one specified sequence, domain, portion or variant thereof. Also disclosed herein are recombinant vectors comprising said anti-IL-17 antibody nucleic acid molecules, host cells containing such nucleic acids and/or recombinant vectors, as well as methods of making and/or using such antibody nucleic acids, vectors and/or host cells.

At least one antibody of the invention binds at least one specified epitope specific to at least one IL-17 protein or variant or derivative such as those provided in at least one of SEQ ID NOS: 1-3. The at least one epitope can comprise at least one antibody binding region that comprises at least one portion of said protein, which epitope is preferably comprised of at least 1-5 amino acids of at least one portion thereof, such as but not limited to, at least one functional, extracellular, soluble, hydrophilic, external or cytoplasmic domain of said protein, or any portion thereof.

The at least one antibody can optionally comprise at least one specified portion of at least one complementarity determining region (CDR) (e.g., CDRI, CDR2 or CDR3 of the heavy or light chain variable region; and optionally further comprising at least one constant or variable framework region or any portion thereof, wherein the antibody blocks, inhibits or prevents at least one activity, such as, but not limited to IL-17 binding to receptor on cell surfaces, IL-17 receptor internalization, IL-17 stimulated Ca2+ mobilization or any other suitable known IL-17 assay. An anti-IL-17 antibody can thus be screened for a corresponding activity according to known methods, such as but not limited to, at least one biological activity towards an IL-17 protein.

Also disclosed herein is at least one IL-17 anti-idiotype antibody to at least one IL-17 antibody of the present invention. The anti-idiotype antibody includes any protein or peptide containing molecule that comprises at least a portion of an immunoglobulin molecule, such as but not limited to at least one ligand binding portion (LBP), such as but not limited to a complementarity determining region (CDR) of a heavy or light chain, or a ligand binding portion thereof, a heavy chain or light chain variable region, a heavy chain or light chain constant region, a framework region, or any portion thereof, that can be incorporated into the anti-idiotype antibody. An anti-idiotype antibody can include or be derived from any mammal, such as but not limited to a human, a mouse, a rabbit, a rat, a rodent, a primate, and the like. Here disclosed are isolated nucleic acid molecules comprising, complementary, or hybridizing to, a polynucleotide encoding at least one IL-17 anti-idiotype antibody, comprising at least one specified sequence, domain, portion or variant thereof.

Also disclosed are recombinant vectors comprising said IL-17 anti-idiotype antibody encoding nucleic acid molecules, host cells containing such nucleic acids and/or recombinant vectors, as well as methods of making and/or using such anti-idiotype antibody nucleic acids, vectors and/or host cells.

Disclosed is at least one method for expressing at least one anti-IL-17 antibody, or IL-17 anti-idiotype antibody, in a host cell, comprising culturing a host cell as described herein under conditions wherein at least one anti-IL-17 antibody is expressed in detectable and/or recoverable amounts.

Disclosed is at least one composition comprising (a) an isolated anti-IL-17 antibody encoding nucleic acid and/or antibody as described herein; and (b) a suitable carrier or diluent. The carrier or diluent can optionally be pharmaceutically acceptable, according to known carriers or diluents. The composition can optionally further comprise at least one further compound, protein or composition.

The present invention further provides at least one anti-IL-17 antibody method or composition, for administering a therapeutically effective amount to modulate or treat at least one IL-17 related condition in a cell, tissue, organ, animal or patient and/or, prior to, subsequent to, or during a related condition, as known in the art and/or as described herein.

Here disclosed are at least one composition, device and/or method of delivery of a therapeutically or prophylactically effective amount of at least one anti-IL-17 antibody,

Disclosed is at least one anti-IL-17 antibody method or composition, for diagnosing at least one IL-17 related condition in a cell, tissue, organ, animal or patient and/or, prior to, subsequent to, or during a related condition, as known in the art and/or as described herein.

Disclosed are at least one composition, device and/or method of delivery for diagnosing of at least one anti-IL-17 antibody, according to the present invention. The at least one antibody can optionally further at least one of: bind IL-17 with an affinity of at least one selected from at least 10⁻⁹ M, at least 10⁻¹⁰ M, at least 10⁻¹¹ M, or at least 10⁻¹² M; substantially neutralize at least one activity of at least one IL-17 protein. Also provided is an isolated nucleic acid encoding at least one isolated mammalian anti-IL-17 antibody; an isolated nucleic acid vector comprising the isolated nucleic acid, and/or a prokaryotic or eukaryotic host cell comprising the isolated nucleic acid. The host cell can optionally be at least one selected from NSO, COS-1, COS-7, HEK293, BHK21, CHO, BSC-1, Hep G2, YB2/0, SP2/0, HeLa, myeloma, or lymphoma cells, or any derivative, immortalized or transformed cell thereof. Also provided is a method for producing at least one anti-IL-17 antibody, comprising translating the antibody encoding nucleic acid under conditions in vitro, in vivo or in situ, such that the IL-17 antibody is expressed in detectable or recoverable amounts.

Also provided is a composition comprising at least one isolated mammalian anti-IL-17 antibody and at least one pharmaceutically acceptable carrier or diluent.

Also disclosed is a method for diagnosing or treating a IL-17 related condition in a cell, tissue, organ or animal, comprising (a) contacting or administering a composition comprising an effective amount of at least one isolated mammalian anti-IL-17 antibody of the invention with, or to, the cell, tissue, organ or animal.

Also disclosed is a medical device, comprising at least one isolated mammalian anti-IL-17 antibody of the invention, wherein the device is suitable to contacting or administering the at least one anti-IL-17 antibody by at least one mode selected from parenteral, subcutaneous, intramuscular, intravenous, intrarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal.

Also disclosed is an article of manufacture for human pharmaceutical or diagnostic use, comprising packaging material and a container comprising a solution, particulate, or a lyophilized form of at least one isolated mammalian anti-IL-17 antibody of the present invention.

Also disclosed is a method for producing at least one isolated mammalian anti-IL-17 antibody of the present invention, comprising providing a host cell or transgenic animal or transgenic plant or plant cell capable of expressing in recoverable amounts the antibody. Further provided in the present invention is at least one anti-IL-17 antibody produced by the above method.

### Brief Descriptions of the Figures

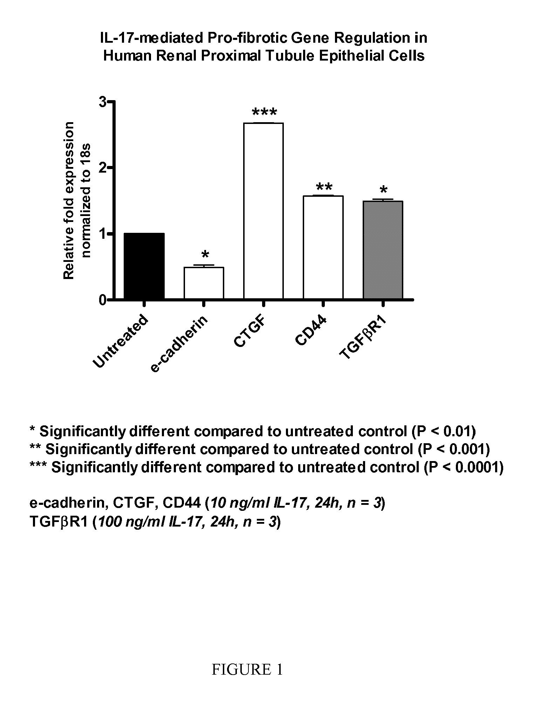

Fig. 1. Effect of IL-17 on pro-fibrotic gene expression in human renal proximal tubule epithelial cells. IL-17 (10 ng/ml) significantly decreased the expression of the pro-epithelial gene e-cadherin (*2.04-fold, P<0.05*) and significantly increased the expression of the pro-fibrotic genes CTGF and CD44 (*2.67 fold, P<0.0001 and 1.57-fold, P<0.001, respectively*). IL-17 (100 ng/ml) also significantly increased TGFβR1 expression (*1.49-fold, P<0.05*). These data indicate IL-17 is capable of eliciting a pro-fibrotic response in human renal proximal tubule epithelium.

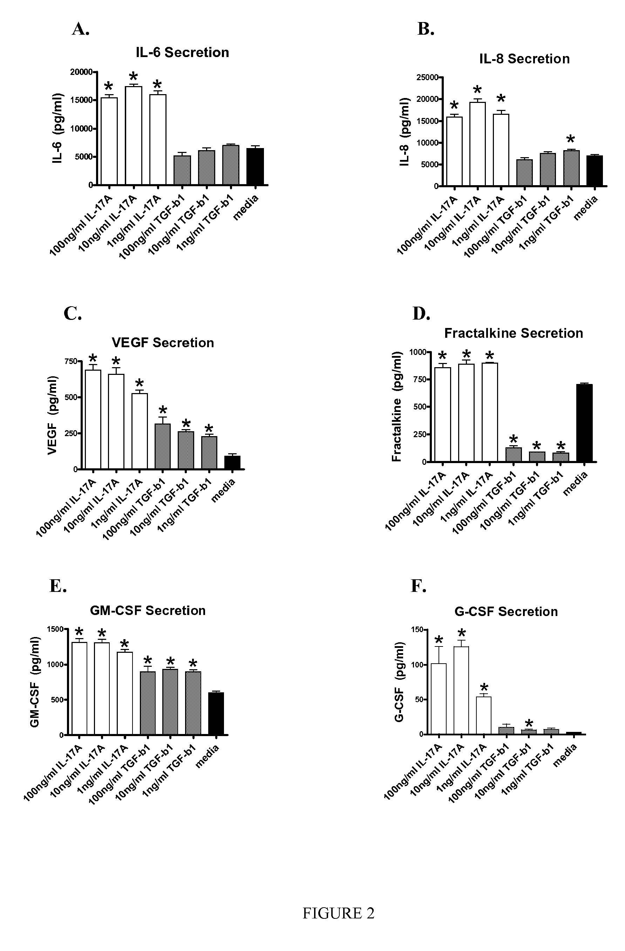

Fig. 2. Effect of IL-17 on the secretion of pro-fibrotic soluble mediators in human renal proximal tubule epithelial cells. IL-17 significantly increased the secretion of IL-6 *(A)*, IL-8 *(B)*, VEGF *(C)*, fractalkine *(D),* GM-CSF *(E)* and G-CSF *(F)* at all concentrations (*1*, *10*, *100 ng*/*ml*, *P<0.05*). For comparison, the effect of TGF-β1 (a well-characterized potent fibrogenic factor) was examined. TGF-β1 significantly stimulated VEGF and GM-CSF secretion at all concentrations however not to the magnitude of IL-17 stimulation. TGF-β1 increased IL-8 and G-CSF secretion at 1 and 10 ng/ml, respectively. There was no effect on IL-6 secretion. Interestingly, TGF-β1 inhibited fractalkine secretion at all concentrations. These data suggest IL-17, in addition to directly promoting a pro-fibrotic phenotypic transition, drives renal inflammation/fibrogenesis by mediating the secretion of soluble pro-inflammatory/fibrotic mediators by the epithelium but may have differential effects from other pro-fibrotic mediators.

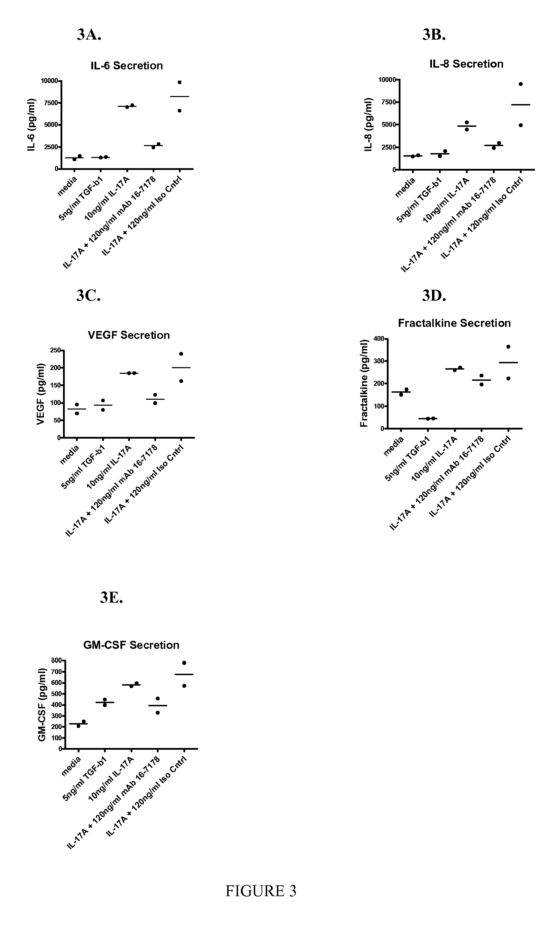

Fig. 3. Antibody-mediated neutralization of IL-17-stimulated pro-fibrotic factor secretion. This preliminary study demonstrates that IL-17-mediated secretion of pro-inflammatory/fibrotic mediators may be inhibited with neutralizing antibody treatment. Pre-incubation of IL-17 (10 ng/ml) with monoclonal anti-IL-17 antibody (120 ng/ml) reduced IL-17-stimulation of IL-6 *(A)*, IL-8 *(B)*, VEGF *(C)*, fractalkine *(D)* and GM-CSF *(E)* secretion by the renal epithelium to levels approaching that of untreated controls. These data indicate the stimulatory effect of IL-17 on the secretion of pro-inflammatory/fibrotic factors is readily inhibited demonstrating IL-17 as a viable target for the attenuation of renal inflammation/fibrosis.

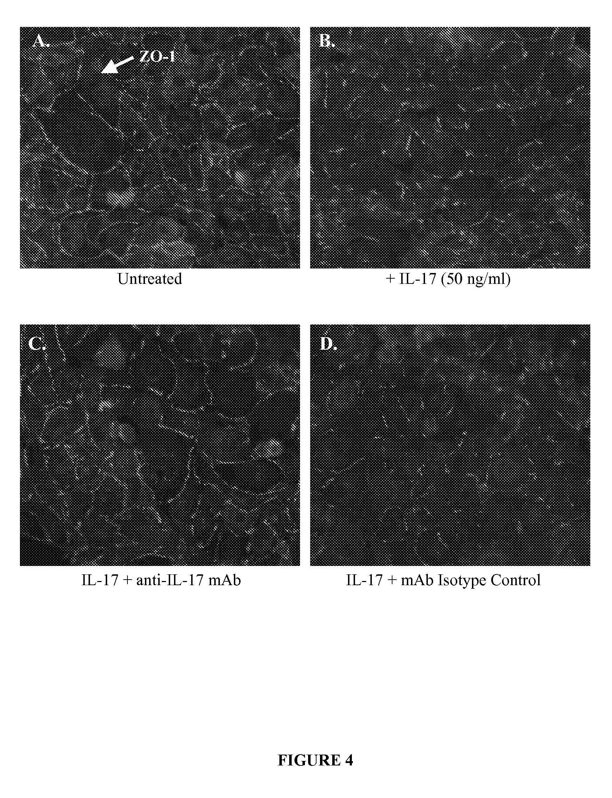

Fig. 4. IL-17-induced downregulation of ZO-1 in human renal proximal tubule epithelial cells and inhibition with neutralizing anti-IL-17 antibody. IL-17 (50 ng/ml) substantially degraded ZO-1 immunoreactivity *(B)* as compared to untreated control cells *(A).* Pre-incubation of IL-17 with a neutralizing anti-IL-17 antibody (0.6 µg/ml) prevented the IL-17-induced downregulation of ZO-1 (C). Pre-incubation of IL-17 with IgG1 isotype control failed to inhibit ZO-1 downregulation (D) indicating the specificity of both IL-17 action and antibody inhibition. (Magnification ∼ 200x).

### DETAILED DESCRIPTION OF THE INVENTION

Here disclosed are methods and compositions for treating IL-17 related conditions in patients having at least one form of fibrosis using IL-17 antagonists, such as small molecule IL-17 antagonists or protein IL-17 antagonists, such as antibodies, including specified portions or variants, specific for at least interleukin-17 (IL-17) protein, such as IL-17A, IL-17F, or muteins thereof (e.g., but not limited to, one of SEQ ID NOS: 1-4), or fragment thereof, including therapeutic formulations, administration and devices.

The present invention provides at least one purified, isolated, recombinant and/or synthetic anti-IL-17 human, primate, rodent, mammalian, chimeric, humanized, engineered, or CDR-grafted, antibodies and IL-17 anti-idiotype antibodies thereto, as well as compositions and encoding nucleic acid molecules comprising at least one polynucleotide encoding at least one anti-IL-17 antibody or anti-idiotype antibody. Disclosed are also methods of making and using such nucleic acids and antibodies and anti-idiotype antibodies, including diagnostic and therapeutic compositions, methods and devices. Citations: Ausubel, et al., ed., Current Protocols in Molecular Biology, John Wiley & Sons, Inc., NY, NY (1987-2007); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2007); Colligan et al., Current Protocols in Protein Science, John Wiley & Sons, NY, NY, (1997-2007).

### Abbreviations

aa: amino acid; BSA: bovine serum albumin; CDR: complementarity-determining regions; ECL: electro-chemiluminescence; HuCAL^{®}: Human Combinatorial Antibody Library; HSA: human serum albumin; IL-17: interleukin-17 ; Ig: Immunoglobulin; IPTG: isopropyl β-D-thiogalactoside; mAb: monoclonal antibody; PBS: phosphate buffered saline, pH 7.4; SET solution equilibrium titration; VH immunoglobulin heavy chain variable region; VL immunoglobulin light chain variable region;

### Definitions

As used herein, an "anti-CCL2 antibody," "anti-IL-17 antibody," "anti-IL-17 antibody portion," or "anti-IL-17 antibody fragment" and/or "anti-IL-17 antibody variant" and the like include any protein or peptide containing molecule that comprises at least a portion of an immunoglobulin molecule, such as but not limited to at least one complementarity determining region (CDR) of a heavy or light chain or a ligand binding portion thereof, a heavy chain or light chain variable region, a heavy chain or light chain constant region, a framework region, or any portion thereof, or at least one portion of an IL-17 receptor or binding protein, which can be incorporated into an antibody of the present invention. Such antibody optionally further affects a specific ligand, such as but not limited to where such antibody modulates, decreases, increases, antagonizes, agonizes, mitigates, alleviates, blocks, inhibits, abrogates and/or interferes with at least one IL-17 activity or binding, or with IL-17 receptor activity or binding, *in vitro*, *in* situ and/or in *vivo*. As a non-limiting example, a suitable anti-IL-17 antibody, specified portion or variant of the present invention can bind at least one IL-17, or specified portions, variants or domains thereof. Non limiting examples of IL-17 protein include, but are not limited to, IL-17A, IL-17F, or muteins thereof (e.g., but not limited to, one of SEQ ID NOS:1-4, or fragments or muteins thereof.

As used herein, "epitope" means a segment or feature of a protein capable of specific binding to an antibody. Epitopes usually consist of chemically active surface groupings of molecules such as amino acids or sugar side chains and usually have specific three-dimensional structural characteristics, as well as specific charge characteristics. Conformational and nonconformational epitopes are distinguished in that the binding to the former but not the latter is lost in the presence of denaturing solvents. Protein epitopes resulting from conformational folding of the IL-17 molecule which arise when amino acids from differing portions of the linear sequence of the IL-17 molecule come together in close proximity in 3-dimensional space are included.

As used herein, the term "human antibody" refers to an antibody in which substantially every part of the protein (e.g., CDR, framework, C_{L}, C_{H} domains (e.g., C_{H}1, C_{H}2, C_{H}3), hinge, (V_{L}, V_{H})) is derived from recombination events of human germline immunoglobulin gene sequences or from mature human antibody sequences. In addition to antibodies isolated humans, such a human antibody may be obtained by immunizing transgenic mice capable of mounting an immune response with human immunoglobulin germline genes (Lonberg et al., Int Rev Immunol 13(1):65-93 (1995) and Fishwald et al., Nat Biotechnol 14(7):845-851 (1996)) or may be selected from a human antibody repertoire library such as described herein. A source of such human gene sequences may be found in any suitable library such as VBASE, a database of human antibody genes (http://www. mrc-cpe.cam.ac.uk/imt-doc) or translated products thereof or at http://people.cryst.bbk.ac.uk/-ubcg07s/ which gives human antibodies classified into groupings based on their amino acid sequence similarities. With the scope of this definition, are composite antibodies or functional fragments of human composite antibodies, which include framework regions from one or more human antibody sequences and CDR regions from two different human or non-human sources. Within the definition of "human antibody" is a composite antibody or functional fragment of a human composite antibody, which contains framework regions from both germline and re-arranged human antibody sequences and CDR regions from two different source antibodies. A human composite antibody or functional fragment of a human composite antibody in accordance with this disclosure includes framework regions from one or more human antibody sequences, and CDR regions derived from a human or non-human antibody sequences or may be entirely synthetic. Thus, a human antibody is distinct from a chimeric or humanized antibody. It is pointed out that a human antibody can be produced by a non-human animal or prokaryotic or eukaryotic cell that is capable of expressing functionally rearranged human immunoglobulin (e.g., heavy chain and/or light chain) genes. Further, when a human antibody is a single chain antibody, it can comprise a linker peptide that is not found in native human antibodies. For example, an Fv can comprise a linker peptide, such as two to about eight glycine or other amino acid residues, which connects the variable region of the heavy chain and the variable region of the light chain. Such linker peptides are considered to be of human origin.

"Humanized" forms of non-human (e.g., murine) antibodies are chimeric antibodies that have substantially replaced sequence portions that were derived from non-human immunoglobulin. For the most part, humanized antibodies are human immunoglobulins (recipient antibody) in which the CDR (the complementarity determining regions which are also known as the hypervariable region) residues of the recipient are replaced by CDR residues from a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues, which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human IgG immunoglobulin. For further details, see Jones et al., Nature 321:522-525 (1986); Reichmann et al., Nature 332:323-329 (1988); and Presta, Curr. Op. Struct. Biol. 2:593-596 (1992).

As used herein, Kd of an antibody refers the dissociation constant, K_{D}, the antibody for a predetermined antigen and is a measure of affinity of the antibody for a specific target. High affinity antibodies have a K_{D} of 10⁻⁸ M or less, more preferably 10⁻⁹ M or less and even more preferably 10⁻¹⁰ M or less, for a predetermined antigen. The term "K_{dis}" or "K_{D}," or "Kd' as used herein, is intended to refer to the dissociation rate of a particular antibody-antigen interaction. The "K_{D}", is the ratio of the rate of dissociation (k₂), also called the "off-rate (k_{off})", to the rate of association rate (k1) or "on-rate (kₒₙ)". Thus, K_{D} equals k₂/k₁ or k_{off} / kₒₙ and is expressed as a molar concentration (M). It follows that the smaller the K_{D}, the stronger the binding. So a K_{D} of 10⁻⁶ M (or 1 microM) indicates weak binding compared to 10⁻⁹ M (or InM).

As used herein, the terms "specificity for" and "specific binding" and "specifically binds" refers to antibody binding to a predetermined antigen with greater affinity than for other antigens or proteins. Typically, the antibody binds with a dissociation constant (K_{D}) of 10⁻⁷ M or less, and binds to the predetermined antigen with a K_{D} that is at least twofold less than its K_{D} for binding to a non-specific antigen (e.g., BSA, casein, or any other specified polypeptide) other than the predetermined antigen. The phrases "an antibody recognizing an antigen" and "an antibody specific for an antigen" are used interchangeably herein with the term "an antibody which binds specifically to an antigen" or "an antigen specific antibody" e.g. a MCP-specific antibody.

As used herein the term IL-17 antagonist small molecule refers to any suitable chemical compound that inhibits IL-17 activity and can be used a potential therapeutic. Such compounds are known in the art, such as indole derivatives, cyclic amine derivatives, ureido derivatives, heterocyclics, anilides, and functional pyrroles with the ability to block CCL2 binding to CCR2B, and/or inhibition of CCR1 or CCL2 itself, as disclosed in PCT publications WO 9905279 (1999), WO 9916876 (1999), WO 9912968, WO 9934818, WO 9909178, WO 9907351, WO 9907678, WO 9940913, WO 9940914, WO 0046195, WO 0046196, WO 0046197, WO 0046198, WO 0046199, WO 9925686, WO 0069815, WO 0069432, WO 9932468, WO 9806703, WO 9904770, WO 99045791.

### 1. Preparation of Antibodies of the Invention

Preparation of human antibodies that are specific for human IL-17 protein or fragments thereof, such as isolated and/or IL-17 protein or a portion thereof (including synthetic molecules, such as synthetic peptides) can be performed using any suitable technique known in the art. Human antibodies can be produced using various techniques known in the art. In one embodiment, the human antibody can be selected from a phage library, where that phage library expresses human antibodies (Vaughan et lo al. Nature Biotechnology 14:309-314 (1996): Sheets et al. PITAS (USA) 95:6157-6162 (1998)); Hoogenboom and Winter, J. Mol. Biol., 227:381 (1991); Marks et al.' J. Mol. Biol., 222:581 (1991)).

Human antibodies can also be made by introducing human immunoglobulin loci into transgenic animals, e.g., mice in which the endogenous immunoglobulin genes have been partially or completely inactivated. For example, a transgenic mouse, comprising a functionally rearranged human immunoglobulin heavy chain transgene and a transgene comprising DNA from a human immunoglobulin light chain locus that can undergo functional rearrangement, can be immunized with human IL-17 or a fragment thereof to elicit the production of antibodies. If desired, the antibody producing cells can be isolated and hybridomas or other immortalized antibody-producing cells can be prepared as described herein and/or as known in the art. Alternatively, the antibody, specified portion or variant can be expressed using the encoding nucleic acid or portion thereof in a suitable host cell.

Upon challenge with an appropriate antigen, human antibody production is observed, which closely resembles that seen in humans in all respects, including gene rearrangement, assembly, and antibody repertoire. This approach is described, for example, in U.S. Patent Nos. 5,545,807; 5,545, 806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huezar, Intern. Rev.

Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol., 147 (1):86-95 (1991); and US Pat No. 5,750,373. Antibody producing cells can also be obtained from the peripheral blood or, preferably the spleen or lymph nodes, of humans or other suitable animals that have been immunized with the antigen of interest. Any other suitable host cell can also be used for expressing heterologous or endogenous nucleic acid encoding an antibody, specified fragment or variant thereof, of the present invention. The fused cells (hybridomas) or recombinant cells can be isolated using selective culture conditions or other suitable known methods, and cloned by limiting dilution or cell sorting, or other known methods. Cells, which produce antibodies with the desired specificity, can be selected by a suitable assay (e.g., ELISA).

In one approach, a hybridoma is produced by fusing a suitable immortal cell line (e.g., a myeloma cell line such as, but not limited to, Sp2/0, Sp2/0-AG14, NSO, NS1, NS2, AE-1, L.5, >243, P3X63Ag8.653, Sp2 SA3, Sp2 MAI, Sp2 SS1, Sp2 SA5, U937, MLA 144, ACT IV, MOLT4, DA-1, JURKAT, WEHI, K-562, COS, RAJI, NIH 3T3, HL-60, MLA 144, NAMAIWA, NEURO 2A, or the like, or heteromylomas, fusion products thereof, or any cell or fusion cell derived there from, or any other suitable cell line as known in the art. See, e.g., www.atcc.org, www.lifetech.com., and the like, with antibody producing cells, such as, but not limited to, isolated or cloned spleen, peripheral blood, lymph, tonsil, or other immune or B cell containing cells, or any other cells expressing heavy or light chain constant or variable or framework or CDR sequences, either as endogenous or heterologous nucleic acid, as recombinant or endogenous, viral, bacterial, algal, prokaryotic, amphibian, insect, reptilian, fish, mammalian, rodent, equine, ovine, goat, sheep, primate, eukaryotic, genomic DNA, cDNA, rDNA, mitochondrial DNA or RNA, chloroplast DNA or RNA, hnRNA, mRNA, tRNA, single, double or triple stranded, hybridized, and the like or any combination thereof. See, e.g., Ausubel, supra, and Colligan, Immunology, supra, chapter 2,

Human antibodies that bind to human IL-17 and that comprise a defined heavy or light chain variable region can be prepared using suitable methods, such as phage display (Katsube, Y., et al., Int J Mol. Med, 1(5):863-868 (1998)). Other suitable methods of producing or isolating antibodies of the requisite specificity can be used, including, but not limited to, methods that select recombinant antibody from a peptide or protein library (e.g., but not limited to, a bacteriophage, ribosome, oligonucleotide, RNA, cDNA, or the like, display library; e.g., as available from Cambridge antibody Technologies, Cambridgeshire, UK; MorphoSys, Martinsreid/Planegg, DE; Biovation, Aberdeen, Scotland, UK; BioInvent, Lund, Sweden; Dyax Corp., Enzon, Affymax/Biosite; Xoma, Berkeley, CA; Ixsys. See, e.g., EP 368,684, PCT/GB91/01134; PCT/GB92/01755; PCT/GB92/002240; PCT/GB92/00883; PCT/GB93/00605; US 08/350260(5/12/94); PCT/GB94/01422; PCT/GB94/02662; PCT/GB97/01835; (CAT/MRC); WO90/14443; WO90/14424; WO90/14430; PCT/US94/1234; WO92/18619; WO96/07754; (Scripps); WO96/13583, WO97/08320 (MorphoSys); WO95/16027 (BioInvent); WO88/06630; WO90/3809 (Dyax); US 4,704,692 (Enzon); PCT/US91/02989 (Affymax); WO89/06283; EP 371 998; EP 550 400; (Xoma); EP 229 046; PCT/US91/07149 (Ixsys); or stochastically generated peptides or proteins - US 5723323, 5763192, 5814476, 5817483, 5824514, 5976862, WO 86/05803, EP 590 689 (Ixsys, now Applied Molecular Evolution (AME), or that rely upon immunization of transgenic animals (e.g., SCID mice, Nguyen et al., Microbiol. Immunol. 41:901-907 (1997); Sandhu et al., Crit. Rev. Biotechnol. 16:95-118 (1996); Eren et al., Immunol. 93:154-161 (1998),

### Other suitable methods of producing antibodies

Other methods for producing the antibodies of the invention that are capable of producing a repertoire of human antibodies, as known in the art and/or as described herein. Such techniques, include, but are not limited to, ribosome display (Hanes et al., Proc. Natl. Acad. Sci. USA, 94:4937-4942 (May 1997); Hanes et al., Proc. Natl. Acad. Sci. USA, 95:14130-14135 (Nov. 1998)); single cell antibody producing technologies (e.g., selected lymphocyte antibody method ("SLAM") (US pat. No. 5,627,052, Wen et al., J. Immunol. 17:887-892 (1987); Babcook et al., Proc. Natl. Acad. Sci. USA 93:7843-7848 (1996)); gel microdroplet and flow cytometry (Powell et al., Biotechnol. 8:333-337 (1990); One Cell Systems, Cambridge, MA; Gray et al., J. Imm. Meth. 182:155-163 (1995); Kenny et al., Bio/Technol. 13:787-790 (1995)); B-cell selection (Steenbakkers et al., Molec. Biol. Reports 19:125-134 (1994); Jonak et al., Progress Biotech, Vol. 5, In Vitro Immunization in Hybridoma Technology, Borrebaeck, ed., Elsevier Science Publishers B.V., Amsterdam, Netherlands (1988)).

Methods for engineering or humanizing non-human or human antibodies can also be used and are well known in the art. Generally, a humanized or engineered antibody has one or more amino acid residues from a source, which is non-human, e.g., but not limited to, mouse, rat, rabbit, non-human primate or other mammal. These human amino acid residues are often referred to as "import" residues, which are typically taken from an "import" variable, constant or other domain of a known human sequence. Known human Ig sequences are well known in the art and can any known sequence. Various strategies for optimizing the binding, conformation, and reduced immunogenicity of engineered humanized antibodies have been described in see e.g. Presta et al. J Immunol. 151:2623-2632, 1993; WO200302019, and WO2005080432.

Such imported sequences can be used to reduce immunogenicity or reduce, enhance or modify binding, affinity, on-rate, off-rate, avidity, specificity, half-life, or any other suitable characteristic, as known in the art. Generally part or all of the non-human or human CDR sequences are maintained while the non-human sequences of the variable and constant regions are replaced with human or other amino acids. Antibodies can also optionally be humanized with retention of high affinity for the antigen and other favorable biological properties. To achieve this goal, humanized antibodies can be optionally prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequences so that the desired antibody characteristic, such as increased affinity for the target antigen(s), is achieved. In general, the CDR residues are directly and most substantially involved in influencing antigen binding. Humanization or engineering of antibodies of the present invention can be performed using any known method, such as but not limited to those described in, Winter (Jones et al., Nature 321:522 (1986); Riechmann et al., Nature 332:323 (1988); Verhoeyen et al., Science 239:1534 (1988)), Sims et al., J. Immunol. 151: 2296 (1993); Chothia and Lesk, J. Mol. Biol. 196:901 (1987), Carter et al., Proc. Natl. Acad. Sci. U.S.A. 89:4285 (1992); Presta et al., J. Immunol. 151:2623 (1993), US patent Nos: 5723323, 5976862, 5824514, 5817483, 5814476, 5763192, 5723323, 5,766886, 5714352, 6204023, 6180370, 5693762, 5530101, 5585089, 5225539; 4816567, PCT/: US98/16280, US96/18978, US91/09630, US91/05939, US94/01234, GB89/01334, GB91/01134, GB92/01755; WO90/14443, WO90/14424, WO90/14430, EP 229246.

Transgenic mice that can produce a repertoire of human antibodies that bind to human antigens can be produced by known methods (e.g., but not limited to, U.S. Pat. Nos: 5,770,428, 5,569,825, 5,545,806, 5,625,126, 5,625,825, 5,633,425, 5,661,016 and 5,789,650 issued to Lonberg et al., Jakobovits et al. WO 98/50433, Jakobovits et al. WO 98/24893, Lonberg et al. WO 98/24884, Lonberg et al. WO 97/13852, Lonberg et al. WO 94/25585, Kucherlapate et al. WO 96/34096, Kucherlapate et al. EP 0463 151 B1, Kucherlapate et al. EP 0710 719 A1, Surani et al. US. Pat. No. 5,545,807, Bruggemann et al. WO 90/04036, Bruggemann et al. EP 0438 474 B1, Lonberg et al. EP 0814 259 A2, Lonberg et al. GB 2 272 440 A, Lonberg et al. Nature 368:856-859 (1994), Taylor et al., Int. Immunol. 6(4)579-591 (1994), Green et al, Nature Genetics 7:13-21 (1994), Mendez et al., Nature Genetics 15:146-156 (1997), Taylor et al., Nucleic Acids Research 20(23):6287-6295 (1992), Tuaillon et al., Proc Natl Acad Sci USA 90(8)3720-3724 (1993), Lonberg et al., Int Rev Immunol 13(1):65-93 (1995) and Fishwald et al., Nat Biotechnol 14(7):845-851 (1996), which are each entirely incorporated herein by reference). Generally, these mice comprise at least one transgene comprising DNA from at least one human immunoglobulin locus that is functionally rearranged, or which can undergo functional rearrangement. The endogenous immunoglobulin loci in such mice can be disrupted or deleted to eliminate the capacity of the animal to produce antibodies encoded by endogenous genes.

Screening antibodies for specific binding to similar proteins or fragments can be conveniently achieved using peptide display libraries. This method involves the screening of large collections of peptides for individual members having the desired function or structure. Antibody screening of peptide display libraries is well known in the art. The displayed peptide sequences can be from 3 to 5000 or more amino acids in length, frequently from 5-100 amino acids long, and often from about 8 to 25 amino acids long. In addition to direct chemical synthetic methods for generating peptide libraries, several recombinant DNA methods have been described. One type involves the display of apeptide sequence on the surface of a bacteriophage or cell. Each bacteriophage or cell contains the nucleotide sequence encoding the particular displayed peptide sequence. Such methods are described in PCT Patent Publication Nos. 91/17271, 91/18980, 91/19818, and 93/08278. Other systems for generating libraries ofpeptides have aspects of both in vitro chemical synthesis and recombinant methods. See, PCT Patent Publication Nos. 92/05258, 92/14843, and 96/19256. See also, U.S. Patent Nos. 5,658,754; and 5,643,768. Peptide display libraries, vector, and screening kits are commercially available from such suppliers as Invitrogen (Carlsbad, CA), and Cambridge antibody Technologies (Cambridgeshire, UK). See, e.g., U.S. Pat. Nos. 4704692, 4939666, 4946778, 5260203, 5455030, 5518889, 5534621, 5656730, 5763733, 5767260, 5856456, assigned to Enzon; 5223409, 5403484, 5571698, 5837500, assigned to Dyax, 5427908, 5580717, assigned to Affymax; 5885793, assigned to Cambridge antibody Technologies; 5750373, assigned to Genentech, 5618920, 5595898, 5576195, 5698435, 5693493, 5698417, assigned to Xoma, Colligan, supra; Ausubel, *supra;* or Sambrook, *supra,*

### 2. Nucleic Acids

Using the information provided herein, a nucleic acid molecule encoding at least one anti-IL-17 antibody can be obtained using methods described herein or as known in the art. Isolated nucleic acid molecules disclosed herein can include nucleic acid molecules comprising an open reading frame (ORF), optionally with one or more introns, e.g., but not limited to, at least one specified portion of at least one CDR, as CDR1, CDR2 and/or CDR3 of at least one heavy chain or light chain nucleic acid molecules comprising the coding sequence for an anti-IL-17 antibody or variable region; and nucleic acid molecules which comprise anucleotide sequence substantially different from those described above but which, due to the degeneracy of the genetic code, still encode at least one anti-IL-17 antibody as described herein and/ or as known in the art. Of course, the genetic code is well known in the art. Thus, it would be routine for one skilled in the art to generate such degenerate nucleic acid variants that code for specific anti-IL-17 antibodies of the present invention. See, e.g., Ausubel, et al., *supra,*

As indicated herein, nucleic acid molecules which comprise a nucleic acid encoding an anti-IL-17 antibody can include, but are not limited to, those encoding the amino acid sequence of an antibody fragment, by itself; the coding sequence for the entire antibody or a portion thereof; the coding sequence for an antibody, fragment or portion, as well as additional sequences, such as the coding sequence of at least one signal leader or fusion peptide, with or without the aforementioned additional coding sequences, such as at least one intron, together with additional, non-coding sequences, including but not limited to, non- coding 5' and 3' sequences, such as the transcribed, non-translated sequences that play a role in transcription, mRNA processing, including splicing and polyadenylation signals (for example - ribosome binding and stability of mRNA); an additional coding sequence that codes for additional amino acids, such as those that provide additional functionalities. Thus, the sequence encoding an antibody can be fused to a marker sequence, such as a sequence encoding a peptide that facilitates purification of the fused antibody comprising an antibody fragment or portion.

### Polynucleotides Which Selectively Hybridize to a Polynucleotide as Described Herein:

The present disclosure provides isolated nucleic acids that hybridize under selective hybridization conditions to a polynucleotide disclosed herein. Thus, these polynucleotides can be used for isolating, detecting, and/or quantifying nucleic acids comprising such polynucleotides. For example, these polynucleotides can be used to identify, isolate, or amplify partial or full-length clones in a deposited library. In some embodiments, the polynucleotides are genomic or cDNA sequences isolated, or otherwise complementary to, a cDNA from a human or mammalian nucleic acid library.

Preferably, the cDNA library comprises at least 80% full-length sequences, preferably at least 85% or 90% full-length sequences, and more preferably at least 95% full-length sequences. The cDNA libraries can be normalized to increase the representation of rare sequences. Low or moderate stringency hybridization conditions are typically, but not exclusively, employed with sequences having a reduced sequence identity relative to complementary sequences. Moderate and high stringency conditions can optionally be employed for sequences of greater identity. Low stringency conditions allow selective hybridization of sequences having about 70% sequence identity and can be employed to identify orthologous or paralogous sequences.

Optionally, polynucleotides will encode at least a portion of an antibody encoded by the polynucleotides described herein. The polynucleotides disclosed embrace nucleic acid sequences that can be employed for selective hybridization to a polynucleotide encoding an antibody of the present invention. See, e.g., Ausubel, supra; Colligan, supra,
Construction of Nucleic Acids: The isolated nucleic acids here disclosed can be made using (a) recombinant methods, (b) synthetic techniques, (c) purification techniques, or combinations thereof, as well-known in the art.
Recombinant Methods for Constructing Nucleic Acids: The isolated nucleic acid compositions such as RNA, cDNA, genomic DNA, or any combination thereof, can be obtained from biological sources using any number of cloning methodologies known to those of skill in the art. In some embodiments, oligonucleotide probes that selectively hybridize, under stringent conditions, to the polynucleotides of the present disclosure are used to identify the desired sequence in a cDNA or genomic DNA library. The isolation of RNA, and construction of cDNA and genomic libraries, is well known to those of ordinary skill in the art. (See, e.g., Ausubel, *supra;* or Sambrook, *supra*)
Nucleic Acid Screening and Isolation Methods: A cDNA or genomic library can be screened using a probe based upon the sequence of a polynucleotide disclosed herein. Probes can be used to hybridize with genomic DNA or cDNA sequences to isolate homologous genes in the same or different organisms. Those of skill in the art will appreciate that various degrees of stringency of hybridization can be employed in the assay; and either the hybridization or the wash medium can be stringent. As the conditions for hybridization become more stringent, there must be a greater degree of complementarity between the probe and the target for duplex formation to occur. The degree of stringency can be controlled by one or more of temperature, ionic strength, pH and the presence of a partially denaturing solvent such as formamide. For example, the stringency of hybridization is conveniently varied by changing the polarity of the reactant solution through, for example, manipulation of the concentration of formamide within the range of 0% to 50%. The degree of complementarity (sequence identity) required for detectable binding will vary in accordance with the stringency of the hybridization medium and/or wash medium. The degree of complementarity will optimally be 100%, or 70- 100%, or any range or value therein. However, it should be understood that minor sequence variations in the probes and primers can be compensated for by reducing the stringency of the hybridization and/or wash medium.
   Methods of amplification of RNA or DNA are well known in the art and can be used without undue experimentation, based on the teaching and guidance presented herein. Known methods of DNA or RNA amplification include, but are not limited to, polymerase chain reaction (PCR) and related amplification processes (Mullis, et al., U.S. Patent No. 4,683,202 (1987); and Innis, et al., PCR Protocols A Guide to Methods and Applications, Eds., Academic Press Inc., San Diego, CA (1990).
Synthetic Methods for Constructing Nucleic Acids: The isolated nucleic acids of the present disclosure can also be prepared by direct chemical synthesis by known methods (see, e.g., Ausubel, et al., supra). Chemical synthesis generally produces a single-stranded oligonucleotide, which can be converted into double-stranded DNA by hybridization with a complementary sequence, or by polymerization with a DNA polymerase using the single strand as a template. One of skill in the art will recognize that while chemical synthesis of DNA can be limited to sequences of about 100 or more bases, longer sequences can be obtained by the ligation of shorter sequences. A particularly preferred method for chemical synthesis of coding sequences is taught in US Patent Nos. 6521427 and 6670127.

### 3. Vectors and Expression Systems

Here disclosed are vectors, preferably, expression vectors, containing anucleic acid encoding the anti-IL-17 antibody, or may be used to obtain plasmids containing various antibody HC or LC genes or portions thereof. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. The present disclosure also relates to vectors that include isolated nucleic acid molecules of the present disclosure, host cells that are genetically engineered with the recombinant vectors, and the production of at least one anti-IL-17 antibody by recombinant techniques, as is well known in the art. See, e.g., Sambrook, et al., supra; Ausubel, et al., supra,

For expression of the antibodies, or antibody fragments thereof, DNAs encoding partial or full-length light and heavy chains, can be inserted into expression cassettes or vectors such that the genes are operatively linked to transcriptional and translational control sequences. A cassette, which encodes an antibody, can be assembled as a construct. A construct can be prepared using methods known in the art. The construct can be prepared as part of a larger plasmid. Such preparation allows the cloning and selection of the correct constructions in an efficient manner. The construct can be located between convenient restriction sites on the plasmid or other vector so that they can be easily isolated from the remaining plasmid sequences.

Generally, a plasmid vector is introduced in a precipitate, such as a calcium phosphate precipitate, or in a complex with a charged lipid of DEAE-dextran. If the vector is a virus, it can be packaged in vitro using an appropriate packaging cell line and then transduced into host cells. Introduction of a vector construct into a host cell can also be effected by electroporation or other known methods. Such methods are described in the art, such as Sambrook, supra, Chapters 1-4 and 16-18; Ausubel, supra, Chapters 1, 9, 13, 15, 16.

In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present).

The light and heavy chain variable regions of the antibodies described herein can be used to create full-length antibody genes of any antibody isotype by inserting them into expression vectors already encoding heavy chain constant and light chain constant regions of the desired isotype such that the VH segment is operatively linked to the CH segment(s) within the vector and the VI, segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody.

In general, a mammalian expression vector will contain (1) regulatory elements, usually in the form of viral promoter or enhancer sequences and characterized by a broad host and tissue range; (2) a "polylinker" sequence, facilitating the insertion of a DNA fragment which comprises the antibody coding sequence within the plasmid vector; and (3) the sequences responsible for intron splicing and polyadenylation of mRNA transcripts. This contiguous region of the promoter-polylinker-polyadenylation site is commonly referred to as the transcription unit. The vector will likely also contain (4) a selectable marker gene(s) (e.g., the beta-lactamase gene), often conferring resistance to an antibiotic (such as ampicillin), allowing selection of initial positive transformants in *E. coli*; and (5) sequences facilitating the replication of the vector in both bacterial and mammalian hosts. A plasmid origin of replication are included for propagation of the expression construct in E. coli and for transient expression in Cos cells, the SV40 origin of replication is included in the expression plasmid.

A promoter may be selected from a SV40 promoter, (e.g., late or early SV40 promoters, the CMV promoter (US Pat.Nos. 5,168,062; 5,385,839), an HSV tk promoter, a pgk (phosphoglycerate kinase) promoter, an EF-1 alpha promoter (US Pat.No. 5,266,491), at least one human immunoglobulin promoter.

Expression vectors will preferably but optionally include at least one selectable marker. Such markers include, e.g., but not limited to, methotrexate (MTX), dihydrofolate reductase (DHFR, US Pat.Nos. 4,399,216; 4,634,665; 4,656,134; 4,956,288; 5,149,636; 5,179,017, ampicillin, neomycin (G418), mycophenolic acid, or glutamine synthetase (GS, US Pat.Nos. 5,122,464; 5,770,359; 5,827,739) resistance for eukaryotic cell culture, and tetracycline or ampicillin resistance genes for culturing in E. coli and other bacteria or prokaryotics. Appropriate culture mediums and conditions for the above-described host cells are known in the art. Suitable vectors will be readily apparent to the skilled artisan.

When eukaryotic host cells are employed, polyadenylation or transcription terminator sequences are typically incorporated into the vector. An example of a terminator sequence is the polyadenylation sequence from the bovine growth hormone gene. Sequences for accurate splicing of the transcript can also be included. An example of a splicing sequence is the VP1 intron from SV40 (Sprague, et al., J. Virol. 45:773-781 (1983)). Additionally, gene sequences to control replication in the host cell can be incorporated into the vector, as known in the art. Also, to avoid high surface expression of heavy chain molecules, it may be necessary to use an expression vector that eliminates transmembrane domain variant splices. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as pIRES1neo, pRetro-Off, pRetro-On, PLXSN, or pLNCX (Clonetech Labs, Palo Alto, CA), pcDNA3.1 (+/-), pcDNA/Zeo (+/-) or pcDNA3.1/Hygro (+/-) (Invitrogen), PSVL and PMSG (Pharmacia, Uppsala, Sweden), pRSVcat (ATCC 37152), pSV2dhfr (ATCC 37146) and pBC12MI (ATCC 67109).

Alternatively, the nucleic acids encoding the antibody sequence can be expressed in stable cell lines that contain the gene integrated into a chromosome. The co-transfection with a selectable marker such as dhfr, gpt, neomycin, or hygromycin allows the identification and isolation of the transfected cells, which express large amounts of the encoded antibody. The DHFR (dihydrofolate reductase) marker is useful to develop cell lines that carry several hundred or even several thousand copies of the gene of interest. Another useful selection marker is the enzyme glutamine synthase (GS) (Murphy, et al., Biochem. J. 227:277-279 (1991); Bebbington, et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of antibodies.

The DNA constructs used in the production of the antibodies of the invention can optionally include at least one insulator sequence. The terms "insulator", "insulator sequence" and "insulator element" are used interchangeably herein. An insulator element is a control element, which insulates the transcription of genes placed within its range of action but which does not perturb gene expression, either negatively or positively. Preferably, an insulator sequence is inserted on either side of the DNA sequence to be transcribed. For example, the insulator can be positioned about 200 bp to about 1 kb, 5' from the promoter, and at least about 1 kb to 5 kb from the promoter, at the 3' end of the gene of interest. The distance of the insulator sequence from the promoter and the 3' end of the gene of interest can be determined by those skilled in the art, depending on the relative sizes of the gene of interest, the promoter and the enhancer used in the construct. In addition, more than one insulator sequence can be positioned 5' from the promoter or at the 3' end of the transgene. For example, two or more insulator sequences can be positioned 5' from the promoter. The insulator or insulators at the 3' end of the transgene can be positioned at the 3' end of the gene of interest, or at the 3'end of a 3' regulatory sequence, e.g., a 3' untranslated region (UTR) or a 3' flanking sequence.

Examples of suitable inducible non-fusion E. coli expression vectors include pTrc (Amann et al., (1988) Gene 69:301-315) and pET 11d (Studier et al., Gene Expression Technology: Methods in Enzymology 185, Academic Press, San Diego, Calif. (1990) 60-89). Target gene expression from the pTrc vector relies on host RNA polymerase transcription from a hybrid trp-lac fusion promoter. Target gene expression from the pET 11d vector relies on transcription from a T7 gn10- lac fusion promoter mediated by a co-expressed viral RNA polymerase (T7 gn1 This viral polymerase is supplied by host strains BL21(DE3) or HMS174(DE3) from a resident λ prophage harboring a T7 gn1 gene under the transcriptional control of the lacUV 5 promoter.

In another embodiment, the expression vector is a yeast expression vector. Examples of vectors for expression in yeast S. cerevisiae include pYepSecl (Baldari et al. (1987) EMBO J. 6:229- 234), pMFa (Kurjan and Herskowitz, (1982) Cell 30:933-943), pJRY88 (Schultz et al. (1987) Gene 54:113-123), pYES2 (Invitrogen Corporation, San Diego, Calif.), and pPicZ (Invitrogen Corp, San Diego, Calif.).

Alternatively, the expression vector is a baculovirus expression vector. Baculovirus vectors available for expression of proteins in cultured insect cells (e.g., Sf9 cells) include the pAc series (Smith et al. (1983) Mol. Cell Biol. 3:2156-2165) and the pVL series (Lucklow and Summers (1989) Virology 170:31-39).

In yet another embodiment, a nucleic acid disclosed herein is expressed in mammalian cells using a mammalian expression vector. Examples of mammalian expression vectors include pCDM8 (Seed (1987) Nature 329:840) and pMT2PC (Kaufman et al. (1987) EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells, see chapters 16 and 17 of Sambrook et al., supra.

In another embodiment, the recombinant mammalian expression vector is capable of directing expression of the nucleic acid, preferentially in a particular cell type, such as lymphoma cells (e.g., mouse myeloma cells). In specific cell types, tissue-specific regulatory elements are used to express the nucleic acid. Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular, promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (e.g., milk whey promoter; U.S. Pat. No. 4,873,316 and European Application Publication No. 264, 166). Developmentally-regulated promoters are also encompassed, for example, by the murine hox promoters (Kessel and Gruss (1990) Science 249:374-379) and the a-fetoprotein promoter (Campes and Tilghman (1989) Genes Dev. 3:537-546).

Also disclosed is a recombinant expression vector comprising a DNA molecule cloned into the expression vector in an antisense orientation. That is, the DNA molecule is operably linked to a regulatory sequence in a manner that allows for expression (by transcription of the DNA molecule) of an RNA molecule that is antisense to the mRNA encoding a polypeptide. Regulatory sequences operably linked to a nucleic acid cloned in the antisense orientation can be chosen which direct the continuous expression of the antisense RNA molecule in a variety of cell types. For instance, viral promoters and/or enhancers, or regulatory sequences can be chosen which direct constitutive, tissue specific, or cell type specific expression of antisense RNA. The antisense expression vector can be in the form of a recombinant plasmid, phagemid, or attenuated virus in which antisense nucleic acids are produced under the control of a high efficiency regulatory region, the activity of which can be determined by the cell type into which the vector is introduced. For a discussion of the regulation of gene expression using antisense genes, see Weintraub et al. (Reviews-- Trends in Genetics, Vol. 1(1) 1986).

### Cloning and Expression in Myeloma Cells

A chimeric mouse/human IgG1k monoclonal antibody against human CD4, known as cM-T412 (EP0511308 was observed to be expressed at high levels in transfected mouse myeloma cells (Looney et al. 1992. Hum Antibodies Hybridomas 3(4):191-200). Without a large effort at optimizing culture conditions, production levels of > 500 mg/L (specific productivity on a pg/cell/day basis not known) were readily obtained at Centocor, Inc. Malvern, PA in 1990. Based on the components of these expression vectors antibody-cloning vectors were developed useful for HC and LC cloning which include the gene promoter/transcription initiation nucleic acid sequence, the 5' untranslated sequences and translation initiation nucleic acid sequences, the nucleic acid sequences encoding the signal sequence, the intron/exon splice donor sequences for the signal intron and the J-C intron, and the J-C intron enhancer nucleic acid sequences. Plasmid p139, a pUC19 plasmid, contains a 5.8 kb EcoRI-EcoRI genomic fragment cloned from C 123 hybridoma cells secreting the fully mouse M-T412 Ab; the fragment contains the promoter and V region part of the cM-T412 HC gene. The starting material for LC V region vector engineering was plasmid p39, a pUC plasmid that contains a 3 kb HindIII-HindIII genomic fragment cloned from C123 hybridoma cells; this fragment contains the promoter and V region part of the cM-T412 LC gene. The engineered vectors derived from p139 and p39 were designed to enable convenient assembly of HC or LC genes suitable for expression in a mammalian host cell in a two-step process that entails 1) cloning DNA encoding a sequence of interest between specially-prepared restriction sites in a V region vector, whereby the V-region coding sequence is positioned immediately downstream of the vector-encoded signal sequence, as well as downstream of part or all of the gene promoter; and 2) transferring a fragment that spans the inserted sequence from the V region vector to the C region vector in the proper orientation whereby the resulting plasmid constitutes the final expression plasmid suitable for expression in cells (Scallon et al. 1995 Cytokine 7(8):759-769).

### Cloning and Expression in CHO Cells

Plasmid pC4 is a derivative of the plasmid pSV2-dhfr (ATCC Accession No. 37146). The plasmid contains the mouse DHFR gene under control of the SV40 early promoter. Chinese hamster ovary- or other cells lacking dihydrofolate activity that are transfected with these plasmids can be selected by growing the cells in a selective medium (e.g., alpha minus MEM, Life Technologies, Gaithersburg, MD) supplemented with the chemotherapeutic agent methotrexate. The amplification of the DHFR genes in cells resistant to methotrexate (MTX) has been well documented (see, e.g., F. W. Alt, et al., J. Biol. Chem. 253:1357-1370 (1978); J. L. Hamlin and C. Ma, Biochem. et Biophys. Acta 1097:107-143 (1990); and M. J. Page and M. A. Sydenham, Biotechnology 9:64-68 (1991)). Cells grown in increasing concentrations of MTX develop resistance to the drug by overproducing the target enzyme, DHFR, as a result of amplification of the DHFR gene. If a second gene is linked to the DHFR gene, it is usually co-amplified and over-expressed. It is known in the art that this approach can be used to develop cell lines carrying more than 1,000 copies of the amplified gene(s). Subsequently, when the methotrexate is withdrawn, cell lines are obtained that contain the amplified gene integrated into one or more chromosome(s) of the host cell.

Plasmid pC4 contains for expressing the gene of interest the strong promoter of the long terminal repeat (LTR) of the Rous Sarcoma Virus (Cullen, et al., Molec. Cell. Biol. 5:438-447 (1985)) plus a fragment isolated from the enhancer of the immediate early gene of human cytomegalovirus (CMV) (Boshart, et al., Cell 41:521-530 (1985)). Downstream of the promoter are BamHI, XbaI, and Asp718 restriction enzyme cleavage sites that allow integration of the genes. Behind these cloning sites the plasmid contains the 3' intron and polyadenylation site of the rat preproinsulin gene. Other high efficiency promoters can also be used for the expression, e.g., the human b-actin promoter, the SV40 early or late promoters or the long terminal repeats from other retroviruses, e.g., HIV and HTLVI. Clontech's Tet-Off and Tet-On gene expression systems and similar systems can be used to express the IL-17 antibody in a regulated way in mammalian cells (M. Gossen, and H. Bujard, Proc. Natl. Acad. Sci. USA 89: 5547-5551 (1992)). For the polyadenylation of the mRNA other signals, e.g., from the human growth hormone or globin genes can be used as well.

### 4. Host Cells for Production of Antibodies

At least one anti-IL-17 antibody of the present invention can be optionally produced by a cell line, a mixed cell line, an immortalized cell or clonal population of immortalized cells, as well known in the art. See, e.g., Ausubel, et al., ed., Current Protocols in Molecular Biology, John Wiley & Sons, Inc., NY, NY (1987-2004); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2004); Colligan et al., Current Protocols in Protein Science, John Wiley & Sons, NY, NY, (1997-2004).

In order to produce biopharmaceutical products, a production cell line capable of efficient and reproducible expression of a recombinant polypeptide(s) is required. The cell line is stable and bankable. A variety of host cell lines can be employed for this purpose. As the understanding of the complexities of how the cellular machinery impact the final amount and composition of a biotherapeutic product, the selection of a host cell line which will impart the needed attributes to the production and the composition of the product become more evident.

Unlike most genes that are transcribed from continuous genomic DNA sequences, antibody genes are assembled from gene segments that may be widely separated in the germ line. In particular, heavy chain genes are formed by recombination of three genomic segments encoding the variable (V), diversity (D) and joining (J)/constant (C) regions of the antibody. Functional light chain genes are formed by joining two gene segments; one encodes the V region and the other encodes the J/C region. Both the heavy chain and kappa light chain loci contain many V gene segments (estimates vary between 100s and 1000s) estimated to span well over 1000 kb. The lambda locus is, by contrast, much smaller and has been shown to span approximately 300 kb on chromosome 16 in the mouse. It consists of two variable gene segments and fourjoining/constant (J/C) region gene segments. Formation of a functional gene requires recombination between a V and a J /*C* element.

In the B-cell in which the antibody is naturally produced, control of transcription of both rearranged heavy and kappa light chain genes depends both on the activity of a tissue specific promoter upstream of the V region and a tissue specific enhancer located in the J-C intron. These elements act synergistically. Also, a second B-cell specific enhancer has been identified in the kappa light chain locus. This further enhancer is located 9 kb downstream of Ckappa· Thus, the hybridoma method of immortalizing antibody expression genes relies on the endogenous promoter and enhancer sequences of the parent B-cell lineage. Alternatively, nucleic acids disclosed herein can be expressed in a host cell by turning on (by manipulation) in a host cell that contains endogenous DNA encoding an antibody of the present invention. Such methods are well known in the art, e.g., as described in US patent Nos. 5,580,734, 5,641,670, 5,733,746, and 5,733,761,

Cloning of antibody genomic DNA into an artificial vector is another method of creating host cells capable of expressing antibodies. However, expression of monoclonal antibodies behind a strong promoter increases the chances of identifying high-producing cell lines and obtaining higher yields of monoclonal antibodies. Antibodies of the invention can be produced in a host cell transfectoma using, for example, a combination of recombinant DNA techniques and gene transfection methods as is well known in the art (e.g., Morrison, S. (1985) Science 229: 1202).

Systems for cloning and expression of a biopharmaceuticals, including antibodies, in a variety of different host cells are well known. Suitable host cells include bacteria, mammalian cells, plant cells, yeast and baculovirus systems and transgenic plants and animals. Mammalian cell lines available in the art for expression of a heterologous polypeptide intact glycosylated proteins include Chinese hamster ovary (CHO) cells, HeLa cells, baby hamster kidney cells (BHK), NSO mouse melanoma cells and derived cell lines, e.g. SP2/0, YB2/0 (ATC CRL- 1662) rat myeloma cells, human embryonic kidney cells (HEK), human embryonic retina cells PerC.6 cells, hep G2 cells, BSC-1 (e.g., ATCC CRL-26) and many others available from, for example, American Type Culture Collection, Manassas, Va (www.atcc.org). A common, preferred bacterial host is E. coli.

Mammalian cells such as CHO cells, myeloma cells, HEK293 cells, BHK cells (BHK21, ATCC CRL-10), mouse Ltk- cells, and NIH3T3 cells have been frequently used for stable expression of heterologous genes. In contrast, cell lines such as Cos (COS-1 ATCC CRL 1650; COS-7, ATCC CRL-1651) and HEK293 are routinely used for transient expression of recombinant proteins.

Preferred mammalian host cells for expressing the recombinant antibodies of the invention include myeloma cells such as Sp2/0, YB2/0 (ATC CRL-1662), NSO, and P3X63.Ag8.653 (e.g. SP2/0-Ag14) because of their high rate of expression. In particular, for use with NSO myeloma cells, another preferred expression system is the GS gene expression system disclosed in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Illustrative of cell cultures useful for the production of the antibodies, specified portions or variants thereof, are mammalian cells. Mammalian cell systems often will be in the form of monolayers of cells although mammalian cell suspensions or bioreactors can also be used.

A number of suitable host cell lines capable of expressing intact glycosylated proteins have been developed in the art, and include the COS-1 (e.g., ATCC CRL 1650), COS-7 (e.g., ATCC CRL-1651), HEK293, BHK21 (e.g., ATCC CRL-10), CHO (e.g., ATCC CRL 1610) and BSC-1 (e.g., ATCC CRL-26) cell lines, Cos-7 cells, CHO cells, hep G2 cells, P3X63Ag8.653, SP2/0-Ag14, 293 cells, HeLa cells and the like, which are readily available from, for example, American Type Culture Collection, Manassas, Va (www.atcc.org). Preferred host cells include cells of lymphoid origin such as myeloma and lymphoma cells. Particularly preferred host cells are P3X63Ag8.653 cells (ATCC Accession Number CRL-1580) and SP2/0-Ag14 cells (ATCC Accession Number CRL-1851).

CHO-K1 and DHFR- CHO cells DG44 and DUK-B11 (G. Urlaub, L.A. Chasin, 1980. Proc. Natl. Acad. Sci. U.S.A. 77, 4216-220) are used for high-level protein production because the amplification of genes of interest is enabled by the incorporation of a selectable, amplifiable marker, DHFR using e.g. the drug methotrexate (MTX) (R.J. Kaufman, 1990. Methods Enzymol. 185: 537-566). DHFR⁻ CHO cells can be successfully used to produce recombinant mAbs at a high level. DHFR⁻ CHO may produce ant-IL-17 antibodies at the rate of 80-110 mg 10⁶ cells⁻¹ day⁻¹ or more than 200 mg 10⁶ cells⁻¹ day⁻¹. A variety of promoters have been used to obtain expression of H- and L-chains in these CHO cells, for example, the b-actin promoter, the human CMV MIE promoter, the Ad virus major late promoter (MLP), the RSV promoter, and a murine leukemia virus LTR. A number of vectors for mAb expression are described in the literature in which the two Ig chains are carried by two different plasmids with an independent selectable/amplifiable marker. Vectors containing one antibody chain, e.g. the H-chain, linked to a DHFR marker, and an L-chain expression cassette with the Neo^{r} marker or vice versa to can be used obtain up to 180 mg of a humanized mAb L⁻¹ 7 day⁻¹ in spinner flasks. The methods used for initial selection and subsequent amplification can be varied and are well known to those skilled in the art. In general, high-level mAb expression can be obtained using the following steps: initial selection and subsequent amplification of candidate clones, coselection (e.g., in cases where both H-chain and L-chain expression vectors carry DHFR expression unit) and amplification, coamplification using different amplifiable markers, and initial selection and amplification in mass culture, followed by dilution cloning to identify individual high-expressing clones. Because integration sites may influence the efficiency of H-chain and L-chain expression and overall mAb expression, single vectors have been created in which the two Ig-chain expression units are placed in tandem. These vectors also carry a dominant selectable marker such as Neo^{r} and the DHFR expression cassette. For a review see Ganguly, S. and A. Shatzman. Expression Systems, mammalian cells IN: Encyclopedia of Bioprocess Technology: Fermentation, Biocatalysis, and Bioseparation. 1999 by John Wiley & Sons, Inc.

Cockett et al. (1990. Bio/Technology 8, 662-667) developed the GS system for high-level expression of heterologous genes in CHO cells. Transfection of an expression vector containing a cDNA (under the transcriptional control of the hCMV promoter) and a GS mini gene (under the control of the SV40 late promoter) into CHO-K1 cells (followed by selection with 20 mM to 500 mM MSX) can be used to yield clones expressing the antibodies of the invention in yields comparable to that of the DHFR- CHO systems. The GS system is discussed in whole or part in connection with European Patent Nos. 0 216 846, 0 256 055, and 0 323 997 and European Patent Application No. 89303964.4.

As a non-limiting example, transgenic tobacco leaves expressing recombinant proteins have been successfully used to provide large amounts of recombinant proteins, e.g., using an inducible promoter. See, e.g., Cramer et al., Curr. Top. Microbiol. Immunol. 240:95-118 (1999) and references cited therein. Also, transgenic maize have been used to express mammalian proteins at commercial production levels, with biological activities equivalent to those produced in other recombinant systems or purified from natural sources. See, e.g., Hood et al., Adv. Exp. Med. Biol. 464:127-147 (1999) and references cited therein. Antibodies have also been produced in large amounts from transgenic plant seeds including antibody fragments, such as single chain antibodies (scFv's), including tobacco seeds and potato tubers. See, e.g., Conrad et al., Plant Mol. Biol. 38:101-109 (1998) and reference cited therein. Thus, antibodies of the present invention can also be produced using transgenic plants, according to know methods. See also, e.g., Fischer et al., Biotechnol. Appl. Biochem. 30:99-108 (Oct., 1999), Ma et al., Trends Biotechnol. 13:522-7 (1995); Ma et al., Plant Physiol. 109:341-6 (1995); Whitelam et al., Biochem. Soc. Trans. 22:940-944 (1994); and references cited therein. See, also generally for plant expression of antibodies, but not limited to, US Pat. No. 5959177.

### 5. Purification of an Antibody

An anti-IL-17 antibody can be recovered and purified from recombinant cell cultures by well-known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxyapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, NY, (1997-2001), e.g., Chapters 1, 4, 6, 8, 9, 10.

Antibodies of the present invention include naturally purified products, products of chemical synthetic procedures, and products produced by recombinant techniques from a eukaryotic host, including, for example, yeast, higher plant, insect and mammalian cells. Depending upon the host employed in a recombinant production procedure, the antibody of the present invention can be glycosylated or can be non-glycosylated, with glycosylated preferred. Such methods are described in many standard laboratory manuals, such as Sambrook, supra, Sections 17.37-17.42; Ausubel, supra, Chapters 10, 12, 13, 16, 18 and 20, Colligan, Protein Science, supra, Chapters 12-14.

### 6. Antibodies of the Invention

Anti-IL-17 antibodies (also termed anti-INTERLEUKIN-17 antibodies or IL-17 antibodies) useful in the methods and compositions of the present invention can optionally be characterized by high affinity binding to IL-17, highly specific binding to IL-17, ability to inhibit one or more of the biologic activities associated with IL-17, and optionally and preferably having low toxicity.

The antibodies of the invention can bind human IL-17 with a wide range of affinities (K_{D}). In a preferred embodiment, at least one human mAb of the present invention can optionally bind human IL-17 with high affinity. For example, a human mAb can bind human IL-17 with a K_{D} equal to or less than about 10⁻⁷ M, such as but not limited to, 0.1-9.9 (or any range or value therein) X 10⁻⁷, 10⁻⁸, 10⁻⁹, 10⁻¹⁰, 10⁻¹¹, 10⁻¹², 10⁻¹³ or any range or value therein.

The affinity or avidity of an antibody for an antigen can be determined experimentally using any suitable method. (See, for example, Berzofsky, et al., "Antibody-Antigen Interactions," In Fundamental Immunology, Paul, W. E., Ed., Raven Press: New York, NY (1984); Kuby, Janis Immunology, W. H. Freeman and Company: New York, NY (1992); and methods described herein). The measured affinity of a particular antibody-antigen interaction can vary if measured under different conditions (e.g., salt concentration, pH). Thus, measurements of affinity and other antigen-binding parameters (e.g., K_{D}, Kₐ, K_{d}) are preferably made with standardized solutions of antibody and antigen, and a standardized buffer, such as the standard solutions and buffers described herein.

The isolated antibodies of the present invention comprise an antibody amino acid sequences disclosed herein encoded by any suitable polynucleotide, or any isolated or prepared antibody. Preferably, the human antibody or antigen-binding fragment binds human IL-17 and, thereby partially or substantially neutralizes at least one biological activity of the protein. An antibody, or specified portion or variant thereof, that partially or preferably substantially neutralizes at least one biological activity of at least one IL-17 protein or fragment can bind the protein or fragment and thereby inhibit activities mediated through the binding of IL-17 to a IL-17 receptor or through other IL-17-dependent or mediated mechanisms. As used herein, the term "neutralizing antibody" refers to an antibody that can inhibit an IL-17-dependent activity by about 20-120%, preferably by at least about 10, 20, 30, 40, 50, 55, 60, 65, 70, 75, 80, 85, 90, 91, 92, 93, 94, 95, 96, 97, 98, 99, 100% or more depending on the assay. The capacity of an anti-IL-17 antibody to inhibit an IL-17-dependent activity is preferably assessed by at least one suitable IL-17 protein or receptor assay, as described herein and/or as known in the art. A human antibody of the invention can be of any class (IgG, IgA, IgM, IgE, IgD, etc.) or isotype and can comprise a kappa or lambda light chain. In one embodiment, the human antibody comprises an IgG heavy chain or defined fragment, for example, at least one of isotypes, IgG1, IgG2, IgG3 or IgG4. Antibodies of this type can be prepared by employing a transgenic mouse or other transgenic non-human mammal comprising at least one human light chain (e.g., IgG, IgA, and IgM (e.g., γ1, γ2, γ3, γ4) transgenes as described herein and/or as known in the art. In another embodiment, the anti-human IL-17 human antibody comprises an IgG1 heavy chain and an IgG1 light chain.

At least one antibody of the invention binds at least one specified epitope specific to at least one IL-17 protein, fragment, portion or any combination thereof. The at least one epitope can comprise at least one antibody binding region that comprises at least one portion of the protein, which epitope is preferably comprised of at least 1-3 amino acids to the entire specified portion of contiguous amino acids of the SEQ ID NOS:1-3.

Generally, the human antibody or antigen-binding fragment of the present invention will comprise an antigen-binding region that comprises at least one human complementarity determining region (CDR1, CDR2 and CDR3) or variant of at least one heavy chain variable region and at least one human complementarity determining region (CDR1, CDR2 and CDR3) or variant of at least one light chain variable region. As a non-limiting example, the antibody or antigen-binding portion or variant can comprise at least one of the heavy chain, and/or a light chain CDR3. In a particular embodiment, the antibody or antigen-binding fragment can have an antigen-binding region that comprises at least a portion of at least one heavy chain CDR (i.e., CDR1, CDR2 and/or CDR3) having the amino acid sequence of the corresponding CDRs 1, 2, and/or 3. In another particular embodiment, the antibody or antigen-binding portion or variant can have an antigen-binding region that comprises at least a portion of at least one light chain CDR (i.e., CDR1, CDR2 and/or CDR3) having the amino acid sequence of the corresponding CDRs 1, 2 and/or 3. Such antibodies can be prepared by chemically joining together the various portions (the CDRs and frameworks) of the antibody using conventional techniques, by preparing and expressing a nucleic acid molecule that encodes the antibody using conventional techniques of recombinant DNA technology or by using any other suitable method.

The anti-IL-17 antibody can comprise at least one of a heavy or light chain variable region having a defined amino acid sequence in the framework regions. For example, in a preferred embodiment, the anti-IL-17 antibody comprises at least one of at least one heavy chain variable region and/or at least one light chain variable region.

Antibody class or isotype (IgA, IgD, IgE, IgG, or IgM) is conferred by the constant regions that are encoded by heavy chain constant region genes. Among human IgG class, there are four subclasses or subtypes: IgG1, IgG2, IgG3 and IgG4 named in order of their natural abundance in serum starting from highest to lowest. IgA antibodies are found as two subclasses, IgA1 and IgA2. As used herein, "isotype switching" also refers to a change between IgG subclasses or subtypes.

The invention also relates to antibodies, antigen-binding fragments, immunoglobulin chains and CDRs comprising amino acids in a sequence that is substantially the same as an amino acid sequence described herein. Preferably, such antibodies or antigen-binding fragments and antibodies comprising such chains or CDRs can bind human IL-17 with high affinity (e.g., K_{D} less than or equal to about 10⁻⁹ M). Amino acid sequences that are substantially the same as the sequences described herein include sequences comprising conservative amino acid substitutions, as well as amino acid deletions and/or insertions. A conservative amino acid substitution refers to the replacement of a first amino acid by a second amino acid that has chemical and/or physical properties (e.g., charge, structure, polarity, hydrophobicity/ hydrophilicity) that are similar to those of the first amino acid. Conservative substitutions include replacement of one amino acid by another within the following groups: lysine (K), arginine (R) and histidine (H); aspartate (D) and glutamate (E); asparagine (N), glutamine (Q), serine (S), threonine (T), tyrosine (Y), K, R, H, D and E; alanine (A), valine (V), leucine (L), isoleucine (I), proline (P), phenylalanine (F), tryptophan (W), methionine (M), cysteine (C) and glycine (G); F, W and Y; C, S and T.

An anti-IL-17 antibody of the present invention can include one or more amino acid substitutions, deletions or additions, either from natural mutations or human manipulation, as specified herein or as taught in Knappik et al. US6828422 for variable regions derived from human germline gene sequences and categorized by sequence similarities into families designated as VH1A, VH1B, VH2, etc. and by light chains as kappa or lambda subgroups. These sequences and other sequences that can be used in the present invention, include, but are not limited to the configurations presented in Table 1, as further described Figures 1-42 of PCT publication WO 05/005604 and US 10/872,932, filed 06/21/2004, entirely incorporated by reference herein, wherein the referenced Figures 1-42 show examples of heavy and light chain variable and constant domain sequences, frameworks, subdomains, regions, and substitutions, portions of which can be used in Ig derived proteins of the present invention, as taught herein.

**Table 1. Human Antibody Configurations**

| | | REGIONS | | | | | | |
|---|---|---|---|---|---|---|---|---|
| Heavy chain variable region | | FR1 | CDR1 | FR2 | CDR2 | FR3 | CDR3 | FR4 |
| Light chain variable region | | FR1 | CDR1 | FR2 | CDR2 | FR3 | CDR3 | FR4 |
| IgAl, IgA2, IgD, IgG1, IgG2, IgG3, IgG4 | Constant Regions | CH1 | Hinge1 -4 | CH2 | CH3 | | | |
| IgA, IgM | | CH1 | Hinge1 -4 | CH2 | CH3 | J-chain | | |
| IgE | | CH1 | | CH2 | CH3 | CH4 | | |

The number of amino acid substitutions a skilled artisan would make depends on many factors, including those described above. Generally speaking, the number of amino acid substitutions, insertions or deletions for any given anti-IL-17 antibody, fragment or variant will not be more than 40, 30, 20, 19, 18, 17, 16, 15, 14, 13, 12, 11, 10, 9, 8, 7, 6, 5, 4, 3, 2, 1, such as 1-30 or any range or value therein, as specified herein.

Amino acids in an anti-IL-17 antibody of the present invention that are essential for function can be identified by methods known in the art, such as site-directed mutagenesis or alanine-scanning mutagenesis (e.g., Ausubel, supra, Chapters 8, 15; Cunningham and Wells, Science 244:1081-1085 (1989)). The latter procedure introduces single alanine mutations at every residue in the molecule. The resulting mutant molecules are then tested for biological activity, such as, but not limited to at least one IL-17 neutralizing activity. Sites that are critical for antibody binding can also be identified by structural analysis such as crystallization, nuclear magnetic resonance or photoaffinity labeling (Smith, et al., J. Mol. Biol. 224:899-904 (1992) and de Vos, et al., Science 255:306-312 (1992)).

As those of skill will appreciate, the present invention includes at least one biologically active antibody of the present invention. Biologically active antibodies have a specific activity at least 20%, 30%, or 40%, and preferably at least 50%, 60%, or 70%, and most preferably at least 80%, 90%, or 95%-1000% of that of the native (non-synthetic), endogenous or related and known antibody. Methods of assaying and quantifying measures of enzymatic activity and substrate specificity, are well known to those of skill in the art and described herein.

In another aspect, the invention relates to human antibodies and antigen-binding fragments, as described herein, which are modified by the covalent attachment of an organic moiety. Such modification can produce an antibody or antigen-binding fragment with improved pharmacokinetic properties (e.g., increased *in vivo* serum half-life). The organic moiety can be a linear or branched hydrophilic polymeric group, fatty acid group, or fatty acid ester group. In particular embodiments, the hydrophilic polymeric group can have a molecular weight of about 800 to about 120,000 Daltons and can be a polyalkane glycol (e.g., polyethylene glycol (PEG), polypropylene glycol (PPG)), carbohydrate polymer, amino acid polymer or polyvinyl pyrolidone, and the fatty acid or fatty acid ester group can comprise from about eight to about forty carbon atoms.

The modified antibodies and antigen-binding fragments of the invention can comprise one or more organic moieties that are covalently bonded, directly or indirectly, to the antibody. Each organic moiety that is bonded to an antibody or antigen-binding fragment of the invention can independently be a hydrophilic polymeric group, a fatty acid group or a fatty acid ester group. As used herein, the term "fatty acid" encompasses mono-carboxylic acids and di-carboxylic acids. A "hydrophilic polymeric group," as the term is used herein, refers to an organic polymer that is more soluble in water than in octane. For example, polylysine is more soluble in water than in octane. Thus, an antibody modified by the covalent attachment of polylysine is encompassed by the invention. Hydrophilic polymers suitable for modifying antibodies of the invention can be linear or branched and include, for example, polyalkane glycols (e.g., PEG, monomethoxy-polyethylene glycol (mPEG), PPG and the like), carbohydrates (e.g., dextran, cellulose, oligosaccharides, polysaccharides and the like), polymers of hydrophilic amino acids (e.g., polylysine, polyarginine, polyaspartate and the like), polyalkane oxides (e.g., polyethylene oxide, polypropylene oxide and the like) and polyvinyl pyrolidone. Preferably, the hydrophilic polymer that modifies the antibody of the invention has a molecular weight of about 800 to about 150,000 Daltons as a separate molecular entity. For example PEG₅₀₀₀ and PEG_{20,000}, wherein the subscript is the average molecular weight of the polymer in Daltons, can be used. The hydrophilic polymeric group can be substituted with one to about six alkyl, fatty acid or fatty acid ester groups. Hydrophilic polymers that are substituted with a fatty acid or fatty acid ester group can be prepared by employing suitable methods. For example, a polymer comprising an amine group can be coupled to a carboxylate of the fatty acid or fatty acid ester, and an activated carboxylate (e.g., activated with N, N-carbonyl diimidazole) on a fatty acid or fatty acid ester can be coupled to a hydroxyl group on a polymer.

Fatty acids and fatty acid esters suitable for modifying antibodies of the invention can be saturated or can contain one or more units of unsaturation. Fatty acids that are suitable for modifying antibodies of the invention include, for example, n-dodecanoate (C₁₂, laurate), n-tetradecanoate (C₁₄, myristate), n-octadecanoate (C₁₈, stearate), n-eicosanoate (C₂₀, arachidate) , n-docosanoate (C₂₂, behenate), n-triacontanoate (C₃₀), n-tetracontanoate (C₄₀), *cis*-Δ9-octadecanoate (C₁₈, oleate), all *cis*-Δ5,8,11,14-eicosatetraenoate (C₂₀, arachidonate), octanedioic acid, tetradecanedioic acid, octadecanedioic acid, docosanedioic acid, and the like. Suitable fatty acid esters include mono-esters of dicarboxylic acids that comprise a linear or branched lower alkyl group. The lower alkyl group can comprise from one to about twelve, preferably one to about six, carbon atoms.

The modified human antibodies and antigen-binding fragments can be prepared using suitable methods, such as by reaction with one or more modifying agents. A "modifying agent" as the term is used herein, refers to a suitable organic group (e.g., hydrophilic polymer, a fatty acid, a fatty acid ester) that comprises an activating group. An "activating group" is a chemical moiety or functional group that can, under appropriate conditions, react with a second chemical group thereby forming a covalent bond between the modifying agent and the second chemical group. For example, amine-reactive activating groups include electrophilic groups such as tosylate, mesylate, halo (chloro, bromo, fluoro, iodo), N-hydroxysuccinimidyl esters (NHS), and the like. Activating groups that can react with thiols include, for example, maleimide, iodoacetyl, acrylolyl, pyridyl disulfides, 5-thiol-2-nitrobenzoic acid thiol (TNB-thiol), and the like. An aldehyde functional group can be coupled to amine- or hydrazide-containing molecules, and an azide group can react with a trivalent phosphorous group to form phosphoramidate or phosphorimide linkages. Suitable methods to introduce activating groups into molecules are known in the art (see for example, Hermanson, G. T., Bioconjugate Techniques, Academic Press: San Diego, CA (1996)). An activating group can be bonded directly to the organic group (e.g., hydrophilic polymer, fatty acid, fatty acid ester), or through a linker moiety, for example a divalent C₁-C₁₂ group wherein one or more carbon atoms can be replaced by a heteroatom such as oxygen, nitrogen or sulfur. Suitable linker moieties include, for example, tetraethylene glycol, -(CH₂)₃-, -NH-(CH₂)₆-NH-, -(CH₂)₂-NH- and -CH₂-O-CH₂-CH₂-O-CH₂-CH₂-O-CH-NH-. Modifying agents that comprise a linker moiety can be produced, for example, by reacting a mono-Boc-alkyldiamine (e.g., mono-Boc-ethylenediamine, mono-Boc-diaminohexane) with a fatty acid in the presence of 1-ethyl-3-(3-dimethylaminopropyl) carbodiimide (EDC) to form an amide bond between the free amine and the fatty acid carboxylate. The Boc protecting group can be removed from the product by treatment with trifluoroacetic acid (TFA) to expose a primary amine that can be coupled to another carboxylate as described, or can be reacted with maleic anhydride and the resulting product cyclized to produce an activated maleimido derivative of the fatty acid. (See, for example, Thompson, et al., WO 92/16221.)

The modified antibodies of the invention can be produced by reacting a human antibody or antigen-binding fragment with a modifying agent. For example, the organic moieties can be bonded to the antibody in a non-site specific manner by employing an amine-reactive modifying agent, for example, an NHS ester of PEG. Modified human antibodies or antigen-binding fragments can also be prepared by reducing disulfide bonds (e.g., intra-chain disulfide bonds) of an antibody or antigen-binding fragment. The reduced antibody or antigen-binding fragment can then be reacted with a thiol-reactive modifying agent to produce the modified antibody of the invention. Modified human antibodies and antigen-binding fragments comprising an organic moiety that is bonded to specific sites of an antibody of the present invention can be prepared using suitable methods, such as reverse proteolysis (Fisch et al., Bioconjugate Chem., 3:147-153 (1992); Werlen et al., Bioconjugate Chem., 5:411-417 (1994); Kumaran et al., Protein Sci. 6(10):2233-2241 (1997); Itoh et al., Bioorg. Chem., 24(1): 59-68 (1996); Capellas et al., Biotechnol. Bioeng., 56(4):456-463 (1997)), and the methods described in Hermanson, G. T., Bioconjugate Techniques, Academic Press: San Diego, CA (1996).

### 7. Anti-Idiotype Antibodies to Anti-IL-17 Antibodies

In addition to monoclonal or chimeric anti-IL-17 antibodies, the present disclosure is also directed to an anti-idiotypic (anti-Id) antibody specific for such antibodies of the invention. An anti-Id antibody is an antibody, which recognizes unique determinants generally associated with the antigen-binding region of another antibody. The anti-Id can be prepared by immunizing an animal of the same species and genetic type (e.g. mouse strain) as the source of the Id antibody with the antibody or a CDR containing region thereof. The immunized animal will recognize and respond to the idiotypic determinants of the immunizing antibody and produce an anti-Id antibody. The anti-Id antibody may also be used as an "immunogen" to induce an immune response in yet another animal, producing a so-called anti-anti-Id antibody.

### 8. Antibody Compositions comprising further therapeutically active ingredients

The composition can optionally further comprise an effective amount of at least one compound or protein selected from at least one of a dermatological drug, an anti-inflammatory drug, an analgesic, a renal drug (e.g., an angiotensin receptor blocker (ARB) or antagonist), an anti-infective drug, a cardiovascular (CV) system drug, a central nervous system (CNS) drug, an autonomic nervous system (ANS) drug, a respiratory tract drug, a gastrointestinal (GI) tract drug, a hormonal drug, a drug for fluid or electrolyte balance, a hematologic drug, an antineoplastic, an immunomodulation drug, an ophthalmic, otic or nasal drug, a topical drug, a nutritional drug or the like. Such drugs are well known in the art, including formulations, indications, dosing and administration for each presented herein (see., e.g., Nursing 2001 Handbook of Drugs, 21st edition, Springhouse Corp., Springhouse, PA, 2001; Health Professional's Drug Guide 2001, ed., Shannon, Wilson, Stang, Prentice-Hall, Inc, Upper Saddle River, NJ; Pharmacotherapy Handbook, Wells et al., ed., Appleton & Lange, Stamford, CT. Anti-IL-17 antibody compositions of the present invention can further comprise at least one of any suitable and effective amount of a composition or pharmaceutical composition comprising at least one anti-IL-17 antibody to a cell, tissue, organ, animal or patient in need of such modulation, treatment or therapy, optionally further comprising at least one selected from at least one TNF antagonist (e.g., but not limited to a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, infliximab, enteracept, CDP-571, CDP-870, certolizumab, afelimomab, lenercept, and the like), an antirheumatic (e.g., methotrexate, auranofin, aurothioglucose, azathioprine, etanercept, gold sodium thiomalate, hydroxychloroquine sulfate, leflunomide, sulfasalzine), a muscle relaxant, a narcotic, a non-steroid anti-inflammatory drug (NSAID), an analgesic, an anesthetic, a sedative, a local anesthetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod, an anabolic steroid, a diabetes related agent, a mineral, a nutritional, a thyroid agent, a vitamin, a calcium related hormone, an antidiarrheal, an antitussive, an antiemetic, an antiulcer, a laxative, an anticoagulant, an erythropoietin (e.g., epoetin alpha), a filgrastim (e.g., G-CSF, Neupogen), a sargramostim (GM-CSF, Leukine), a chronic obstructive pulmonary disease (COPD) agent, an anti-fibrotic agent, an immunization, an immunoglobulin, an immunosuppressive (e.g., basiliximab, cyclosporine, daclizumab), a growth hormone, a hormone replacement drug, an estrogen receptor modulator, a mydriatic, a cycloplegic, an alkylating agent, an antimetabolite, a mitotic inhibitor, a radiopharmaceutical, an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, dornase alpha (Pulmozyme(TM)), a cytokine or a cytokine antagonist. Non-limiting examples of such cytokines include, but are not limited to, any of IL-1 to IL-29. Suitable dosages are well known in the art. See, e.g., Wells et al., eds., Pharmacotherapy Handbook, 2nd Edition, Appleton and Lange, Stamford, CT (2000); PDR Pharmacopoeia, Tarascon Pocket Pharmacopoeia 2000, Deluxe Edition, Tarascon Publishing, Loma Linda, CA (2000).

Such anti-cancer or anti-infectives can also include toxin molecules that are associated, bound, co-formulated or co-administered with at least one antibody of the present invention. The toxin can optionally act to selectively kill the pathologic cell or tissue. The pathologic cell can be a cancer or other cell. Such toxins can be, but are not limited to, purified or recombinant toxin or toxin fragment comprising at least one functional cytotoxic domain of toxin, e.g., selected from at least one of ricin, diphtheria toxin, a venom toxin, or a bacterial toxin. The term toxin also includes both endotoxins and exotoxins produced by any naturally occurring, mutant or recombinant bacteria or viruses which may cause any pathological condition in humans and other mammals, including toxin shock, which can result in death. Such toxins may include, but are not limited to, enterotoxigenic *E. coli* heat-labile enterotoxin (LT), heat-stable enterotoxin (ST), *Shigella* cytotoxin, *Aeromonas* enterotoxins, toxic shock syndrome toxin-1 (TSST-1), Staphylococcal enterotoxin A (SEA), B (SEB), or C (SEC), Streptococcal enterotoxins and the like. Such bacteria include, but are not limited to, strains of a species of enterotoxigenic *E. coli* (ETEC), enterohemorrhagic *E. coli* (e.g., strains of serotype 0157:H7), Staphylococcus species (e.g., *Staphylococcus aureus, Staphylococcus pyogenes*), *Shigella* species (e.g., *Shigella dysenteriae, Shigella flexneri, Shigella boydii, and Shigella sonnei), Salmonella* species (e.g., *Salmonella typhi, Salmonella cholera-suis, Salmonella enteritidis*), *Clostridium* species (e.g., *Clostridium perfingens, Clostridium dificile, Clostridium botulinum*), *Camphlobacter* species (e.g., *Camphlobacter jejuni, Camphlobacter fetus*), *Heliobacter* species, (e.g., *Heliobacter pylori*), *Aeromonas* species (e.g., *Aeromonas sobria, Aeromonas hydrophila, Aeromonas caviae*), *Pleisomonas shigelloides, Yersina enterocolitica, Vibrios* species (e.g., *Vibrios cholerae, Vibrios parahemolyticus*), *Klebsiella* species, *Pseudomonas aeruginosa,* and *Streptococci.* See, e.g., Stein, ed., INTERNAL MEDICINE, 3rd ed., pp 1-13, Little, Brown and Co., Boston, (1990); Evans et al., eds., Bacterial Infections of Humans: Epidemiology and Control, 2d. Ed., pp 239-254, Plenum Medical Book Co., New York (1991); Mandell et al, Principles and Practice of Infectious Diseases, 3d. Ed., Churchill Livingstone, New York (1990); Berkow et al, eds., The Merck Manual, 16th edition, Merck and Co., Rahway, N.J., 1992; Wood et al, FEMS Microbiology Immunology, 76:121-134 (1991); Marrack et al, Science, 248:705-711 (1990). Anti-IL-17 antibody compounds, compositions or combinations of the present invention can further comprise at least one of any suitable auxiliary agent, such as, but not limited to, diluent, binder, stabilizer, buffers, salts, lipophilic solvents, preservative, adjuvant or the like. Pharmaceutically acceptable auxiliaries are preferred. Non-limiting examples of, and methods of preparing such sterile solutions are well known in the art, such as, but limited to, Gennaro, Ed., Remington's Pharmaceutical Sciences, 18th Edition, Mack Publishing Co. (Easton, PA) 1990. Pharmaceutically acceptable carriers can be routinely selected that are suitable for the mode of administration, solubility and/or stability of the anti-IL-17 antibody, fragment or variant composition as well known in the art or as described herein.

Pharmaceutical excipients and additives useful in the present composition include but are not limited to proteins, peptides, amino acids, lipids, and carbohydrates (e.g., sugars, including monosaccharides, di-, tri-, tetra-, and oligosaccharides; derivatized sugars such as alditols, aldonic acids, esterified sugars and the like; and polysaccharides or sugar polymers), which can be present singly or in combination, comprising alone or in combination 1-99.99% by weight or volume. Exemplary protein excipients include serum albumin such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. One preferred amino acid is glycine.

Carbohydrate excipients suitable for use in the invention include, for example, monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), myoinositol and the like. Preferred carbohydrate excipients for use in the present invention are mannitol, trehalose, and raffinose.

Anti-IL-17 antibody compositions can also include a buffer or a pH-adjusting agent; typically, the buffer is a salt prepared from an organic acid or base. Representative buffers include organic acid salts such as salts of citric acid, ascorbic acid, gluconic acid, carbonic acid, tartaric acid, succinic acid, acetic acid, or phthalic acid; Tris, tromethamine hydrochloride, or phosphate buffers. Preferred buffers for use in the present compositions are organic acid salts such as citrate.

Additionally, anti-IL-17 antibody compositions of the invention can include polymeric excipients/additives such as polyvinylpyrrolidones, ficolls (a polymeric sugar), dextrates (e.g., cyclodextrins, such as 2-hydroxypropyl-β-cyclodextrin), polyethylene glycols, flavoring agents, antimicrobial agents, sweeteners, antioxidants, antistatic agents, surfactants (e.g., polysorbates such as "TWEEN 20" and "TWEEN 80"), lipids (e.g., phospholipids, fatty acids), steroids (e.g., cholesterol), and chelating agents (e.g., EDTA).

These and additional known pharmaceutical excipients and/or additives suitable for use in the anti-IL-17 antibody, portion or variant compositions according to the invention are known in the art, e.g., as listed in "Remington: The Science & Practice of Pharmacy", 19th ed., Williams & Williams, (1995), and in the "Physician's Desk Reference", 52nd ed., Medical Economics, Montvale, NJ (1998), the disclosures of which are entirely incorporated herein by reference. Preferred carrier or excipient materials are carbohydrates (e.g., saccharides and alditols) and buffers (e.g., citrate) or polymeric agents.

### 9. Formulations

As noted above, here disclosed are stable formulations suitable for pharmaceutical or veterinary use, comprising at least one anti-IL-17 antibody in a pharmaceutically acceptable formulation.

As noted above, disclosed is an article of manufacture, comprising packaging material and at least one vial comprising a solution of at least one anti-IL-17 antibody with the prescribed buffers and/or preservatives, optionally in an aqueous diluent, wherein said packaging material comprises a label that indicates that such solution can be held over a period of 1, 2, 3, 4, 5, 6, 9, 12, 18, 20, 24, 30, 36, 40, 48, 54, 60, 66, 72 hours or greater. Also disclosed is an article of manufacture, comprising packaging material, a first vial comprising lyophilized at least one anti-IL-17 antibody, and a second vial comprising an aqueous diluent of prescribed buffer or preservative, wherein said packaging material comprises a label that instructs a patient to reconstitute the at least one anti-IL-17 antibody in the aqueous diluent to form a solution that can be held over a period of twenty-four hours or greater.

The range of at least one anti-IL-17 antibody in the product here disclosed includes amounts yielding upon reconstitution, if in a wet/dry system, concentrations from about 1.0 µg/ml to about 1000 mg/ml, although lower and higher concentrations are operable and are dependent on the intended delivery vehicle, e.g., solution formulations will differ from transdermal patch, pulmonary, transmucosal, or osmotic or micro pump methods.

The aqueous diluent optionally further comprises a pharmaceutically acceptable preservative. Preferred preservatives include those selected from the group consisting of phenol, m-cresol, p-cresol, o-cresol, chlorocresol, benzyl alcohol, alkylparaben (methyl, ethyl, propyl, butyl and the like), benzalkonium chloride, benzethonium chloride, sodium dehydroacetate and thimerosal, or mixtures thereof. The concentration of preservative used in the formulation is a concentration sufficient to yield an anti-microbial effect. Such concentrations are dependent on the preservative selected and are readily determined by the skilled artisan.

Other excipients, e.g., isotonicity agents, buffers, antioxidants, preservative enhancers, can be optionally and preferably added to the diluent. An isotonicity agent, such as glycerin, is commonly used at known concentrations. A physiologically tolerated buffer is preferably added to provide improved pH control. The formulations can cover a wide range of pHs, such as from about pH 4 to about pH 10, and preferred ranges from about pH 5 to about pH 9, and a most preferred range of about 6.0 to about 8.0. Preferably the formulations of the present invention have pH between about 6.8 and about 7.8. Preferred buffers include phosphate buffers, most preferably sodium phosphate, particularly phosphate buffered saline (PBS). Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), and PEG (polyethylene glycol) or non-ionic surfactants such as polysorbate 20 or 80 or poloxamer 184 or 188, Pluronic® polyls, other block co-polymers, and chelators such as EDTA and EGTA can optionally be added to the formulations or compositions to reduce aggregation. These additives are particularly useful if a pump or plastic container is used to administer the formulation. The presence of pharmaceutically acceptable surfactant mitigates the propensity for the protein to aggregate.

The formulations here disclosed can be prepared by a process, which comprises mixing at least one anti-IL-17 antibody and a buffered solution in quantities sufficient to provide the protein at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

Said formulations can be provided to patients as solutions or as dual vials comprising a vial of lyophilized at least one anti-IL-17 antibody that is reconstituted with a second vial containing water, a preservative and/or excipients, preferably a phosphate buffer and/or saline and a chosen salt, in an aqueous diluent. Either a single solution vial or dual vial requiring reconstitution can be reused multiple times and can suffice for a single or multiple cycles of patient treatment and thus can provide a more convenient treatment regimen than currently available.

The present articles of manufacture are useful for administration over a period of immediately to twenty-four hours or greater. Accordingly, the presently articles of manufacture offer significant advantages to the patient. Formulations can optionally be safely stored at temperatures of from about 2 to about 40°C and retain the biologically activity of the protein for extended periods of time, thus, allowing a package label indicating that the solution can be held and/or used over a period of 6, 12, 18, 24, 36, 48, 72, or 96 hours or greater. If preserved diluent is used, such label can include use up to 1-12 months, one-half, one and a half, and/or two years.

The products can be provided indirectly to patients by providing to pharmacies, clinics, or other such institutions and facilities, clear solutions or dual vials comprising a vial of lyophilized at least one anti-IL-17 antibody that is reconstituted with a second vial containing the aqueous diluent. The clear solution in this case can be up to one liter or even larger in size, providing a large reservoir from which smaller portions of the at least one antibody solution can be retrieved one or multiple times for transfer into smaller vials and provided by the pharmacy or clinic to their customers and/or patients.

Recognized devices comprising these single vial systems include those pen-injector devices for delivery of a solution such as BD Pens, BD Autojector^{®}, Humaject^{®,} NovoPen^{®}, B-D^{®}Pen, AutoPen^{®}, and OptiPen^{®}, GenotropinPen^{®}, Genotronorm Pen^{®}, Humatro Pen^{®}, Reco-Pen^{®}, Roferon Pen^{®}, Biojector^{®}, Iject^{®}, J-tip Needle-Free Injector^{®}, Intraject^{®}, Medi-Ject^{®}, e.g., as made or developed by Becton Dickensen (Franklin Lakes, NJ, www.bectondickenson.com), Disetronic (Burgdorf, Switzerland, www.disetronic.com; Bioject, Portland, Oregon (www.bioject.com); National Medical Products , Weston Medical (Peterborough, UK, www.weston-medical.com), Medi-Ject Corp (Minneapolis, MN, www.mediject.com). Recognized devices comprising a dual vial system include those pen-injector systems for reconstituting a lyophilized drug in a cartridge for delivery of the reconstituted solution such as the HumatroPen^{®}.

The products here disclosed include packaging material. The packaging material provides, in addition to the information required by the regulatory agencies, the conditions under which the product can be used. The packaging material provides instructions to the patient to reconstitute the at least one anti-IL-17 antibody in the aqueous diluent to form a solution and to use the solution over a period of 2-24 hours or greater for the two vial, wet/dry, product. For the single vial, solution product, the label indicates that such solution can be used over a period of 2-24 hours or greater. Said products are useful for human pharmaceutical product use.

Other formulations or methods of stabilizing the anti-IL-17 antibody may result in other than a clear solution of lyophilized powder comprising said antibody. Among non-clear solutions are formulations comprising particulate suspensions, said particulates being a composition containing the anti-IL-17 antibody in a structure of variable dimension and known variously as a microsphere, micro particle, nanoparticle, nanosphere, or liposome. Such relatively homogenous essentially spherical particulate formulations containing an active agent can be formed by contacting an aqueous phase containing the active and a polymer and a nonaqueous phase followed by evaporation of the nonaqueous phase to cause the coalescence of particles from the aqueous phase as taught in U.S. 4,589,330. Porous micro particles can be prepared using a first phase containing active and a polymer dispersed in a continuous solvent and removing said solvent from the suspension by freeze-drying or dilution-extraction-precipitation as taught in U.S. 4,818,542. Preferred polymers for such preparations are natural or synthetic copolymers or polymer selected from the group consisting of gelatin agar, starch, arabinogalactan, albumin, collagen, polyglycolic acid, polylactic aced, glycolide-L(-) lactide poly(episilon-caprolactone, poly(epsilon-caprolactone-CO-lactic acid), poly(epsilon-caprolactone-CO-glycolic acid), poly(β-hydroxy butyric acid), polyethylene oxide, polyethylene, poly(alkyl-2-cyanoacrylate), poly(hydroxyethyl methacrylate), polyamides, poly(amino acids), poly(2-hydroxyethyl DL-aspartamide), poly(ester urea), poly(L-phenylalanine/ethylene glycol/1,6-diisocyanatohexane) and poly(methyl methacrylate). Particularly preferred polymers are polyesters such as polyglycolic acid, polylactic aced, glycolide-L(-) lactide poly(episilon-caprolactone, poly(epsilon-caprolactone-CO-lactic acid), and poly(epsilon-caprolactone-CO-glycolic acid. Solvents useful for dissolving the polymer and/or the active include: water, hexafluoroisopropanol, methylenechloride, tetrahydrofuran, hexane, benzene, or hexafluoroacetone sesquihydrate. The process of dispersing the active containing phase with a second phase may include pressure forcing said first phase through an orifice in a nozzle to affect droplet formation.

Dry powder formulations may result from processes other than lyophilization such as by spray drying or solvent extraction by evaporation or by precipitation of a crystalline composition followed by one or more steps to remove aqueous or nonaqueous solvent. Preparation of a spray-dried antibody preparation is taught in U.S. 6,019,968. The antibody-based dry powder compositions may be produced by spray drying solutions or slurries of the antibody and, optionally, excipients, in a solvent under conditions to provide a respirable dry powder. Solvents may include polar compounds such as water and ethanol, which may be readily dried. Antibody stability may be enhanced by performing the spray drying procedures in the absence of oxygen, such as under a nitrogen blanket or by using nitrogen as the drying gas. Another relatively dry formulation is a dispersion of a plurality of perforated microstructures dispersed in a suspension medium that typically comprises a hydrofluoroalkane propellant as taught in WO 9916419. The stabilized dispersions may be administered to the lung of a patient using a metered dose inhaler. Equipment useful in the commercial manufacture of spray dried medicaments are manufactured by Buchi Ltd. or Niro Corp.

At least one anti-IL-17 antibody in either the stable or preserved formulations or solutions described herein, can be administered to a patient in accordance with the present invention via a variety of delivery methods including SC or IM injection; transdermal, pulmonary, transmucosal, implant, osmotic pump, cartridge, micro pump, or other means appreciated by the skilled artisan, as well-known in the art.

### 10. Therapeutic Applications.

Disclosed is a method for modulating or treating at least one IL-17 related disease, in a cell, tissue, organ, animal, or patient, as known in the art or as described herein, using at least one IL-17 antibody of the present invention. The present disclosure also provides a method for modulating or treating at least one IL-17 related disease, in a cell, tissue, organ, animal, or patient including, but not limited to, at least one of malignant disease, metabolic disease, an immune or inflammatory related disease, a cardiovascular disease, an infectious disease, or a neurological disease.

Such conditions are selected from, but not limited to, diseases or conditions mediated by cell adhesion and/or angiogenesis. Such diseases or conditions include an immune disorder or disease, a cardiovascular disorder or disease, an infectious, malignant, and/or neurologic disorder or disease, or other known or specified IL-17related conditions. In particular, the antibodies are useful for the treatment of diseases that involve angiogenesis such as disease of the eye and neoplastic disease, tissue remodeling such as restenosis, and proliferation of certain cells types particularly epithelial and squamous cell carcinomas. Particular indications include use in the treatment of atherosclerosis, restenosis, cancer metastasis, rheumatoid arthritis, diabetic retinopathy and macular degeneration. The neutralizing antibodies of the invention are also useful to prevent or treat unwanted bone resorption or degradation, for example as found in osteoporosis or resulting from PTHrP overexpression by some tumors. The antibodies may also be useful in the treatment of various fibrotic diseases such as idiopathic pulmonary fibrosis, diabetic nephropathy, hepatitis, and cirrhosis.

Thus, here disclosed is a method for modulating or treating at least one IL-17 related disease, in a cell, tissue, organ, animal, or patient, as known in the art or as described herein, using at least one IL-17 antibody of the present invention. Particular indications are discussed below:

### Pulmonary Disease

The present disclosure also provides a method for modulating or treating at least one malignant disease in a cell, tissue, organ, animal or patient, including, but not limited to, at least one of: pneumonia; lung abscess; occupational lung diseases caused be agents in the form or dusts, gases, or mists; asthma, bronchiolitis fibrosa obliterans, respiratory failure, hypersensitivity diseases of the lungs including hypersensitivity pneumonitis (extrinsic allergic alveolitis), allergic bronchopulmonary aspergillosis, and drug reactions; adult respiratory distress syndrome (ARDS), Goodpasture's Syndrome, chronic obstructive airway disorders (COPD), idiopathic interstitial lung diseases such as idiopathic pulmonary fibrosis and sarcoidosis, desquamative interstitial pneumonia, acute interstitial pneumonia, respiratory bronchiolitis-associated interstitial lung disease, idiopathic bronchiolitis obliterans with organizing pneumonia, lymphocytic interstitial pneumonitis, Langerhans' cell granulomatosis, idiopathic pulmonary hemosiderosis; acute bronchitis, pulmonary alveolar proteinosis, bronchiectasis, pleural disorders, atelectasis, cystic fibrosis, and tumors of the lung, and pulmonary embolism.

### Malignant Disease

The present disclosure also provides a method for modulating or treating at least one malignant disease in a cell, tissue, organ, animal or patient, including, but not limited to, at least one of: leukemia, acute leukemia, acute lymphoblastic leukemia (ALL), B-cell, T-cell or FAB ALL, acute myeloid leukemia (AML), chromic myelocytic leukemia (CML), chronic lymphocytic leukemia (CLL), hairy cell leukemia, myelodyplastic syndrome (MDS), a lymphoma, Hodgkin's disease, a malignant lymphoma, non-Hodgkin's lymphoma, Burkitt's lymphoma, multiple myeloma, Kaposi's sarcoma, colorectal carcinoma, pancreatic carcinoma, renal cell carcinoma, breast cancer, nasopharyngeal carcinoma, malignant

histiocytosis, paraneoplastic syndrome/hypercalcemia of malignancy, solid tumors, adenocarcinomas, squamous cell carcinomas, sarcomas, malignant melanoma, particularly metastatic melanoma, hemangioma, metastatic disease, cancer related bone resorption, cancer related bone pain, and the like.

### Immune Related Disease

The present disclosure also provides a method for modulating or treating at least one immune related disease, in a cell, tissue, organ, animal, or patient including, but not limited to, at least one of rheumatoid arthritis, juvenile rheumatoid arthritis, systemic onset juvenile rheumatoid arthritis, psoriatic arthritis, ankylosing spondilitis, gastric ulcer, seronegative arthropathies, osteoarthritis, inflammatory bowel disease, ulcerative colitis, systemic lupus erythematosis, antiphospholipid syndrome, iridocyclitis/uveitis/optic neuritis, idiopathic pulmonary fibrosis, systemic vasculitis/Wegener's granulomatosis, sarcoidosis, orchitis/vasectomy reversal procedures, allergic/atopic diseases, asthma, allergic rhinitis, eczema, allergic contact dermatitis, allergic conjunctivitis, hypersensitivity pneumonitis, transplants, organ transplant rejection, graft-versus-host disease, systemic inflammatory response syndrome, sepsis syndrome, gram positive sepsis, gram negative sepsis, culture negative sepsis, fungal sepsis, neutropenic fever, urosepsis, meningococcemia, trauma/hemorrhage, burns, ionizing radiation exposure, acute pancreatitis, adult respiratory distress syndrome, rheumatoid arthritis, alcohol-induced hepatitis, chronic inflammatory pathologies, sarcoidosis, Crohn's pathology, sickle cell anemia, diabetes, nephrosis, atopic diseases, hypersensitity reactions, allergic rhinitis, hay fever, perennial rhinitis, conjunctivitis, endometriosis, asthma, urticaria, systemic anaphylaxis, dermatitis, pernicious anemia, hemolytic diseases, thrombocytopenia, graft rejection of any organ or tissue, kidney transplant rejection, heart transplant rejection, liver transplant rejection, pancreas transplant rejection, lung transplant rejection, bone marrow transplant (BMT) rejection, skin allograft rejection, cartilage transplant rejection, bone graft rejection, small bowel transplant rejection, fetal thymus implant rejection, parathyroid transplant rejection, xenograft rejection of any organ or tissue, allograft rejection, anti-receptor hypersensitivity reactions, Graves disease, Raynoud's disease, type B insulin-resistant diabetes, asthma, myasthenia gravis, antibody-meditated cytotoxicity, type III hypersensitivity reactions, systemic lupus erythematosis, POEMS syndrome (polyneuropathy, organomegaly, endocrinopathy, monoclonal gammopathy, and skin changes syndrome), polyneuropathy, organomegaly, endocrinopathy, monoclonal gammopathy, skin changes syndrome, antiphospholipid syndrome, pemphigus, scleroderma, mixed connective tissue disease, idiopathic Addison's disease, diabetes mellitus, chronic active hepatitis, primary biliary cirrhosis, vitiligo, vasculitis, post-MI cardiotomy syndrome, type IV hypersensitivity , contact dermatitis, hypersensitivity pneumonitis, allograft rejection, granulomas due to intracellular organisms, drug sensitivity, metabolic/idiopathic, Wilson's disease, hemachromatosis, alpha-1-antitrypsin deficiency, diabetic retinopathy, Hashimoto's thyroiditis, osteoporosis, hypothalamic-pituitary-adrenal axis evaluation, primary biliary cirrhosis, thyroiditis, encephalomyelitis, cachexia, cystic fibrosis, neonatal chronic lung disease, chronic obstructive pulmonary disease (COPD), familial hematophagocytic lymphohistiocytosis, dermatologic conditions, psoriasis, alopecia, nephrotic syndrome, nephritis, glomerular nephritis, acute renal failure, hemodialysis, uremia, toxicity, preeclampsia, OKT3 therapy, anti-CD3 therapy, cytokine therapy, chemotherapy, radiation therapy (e.g., including but not limited toasthenia, anemia, cachexia, and the like), chronic salicylate intoxication, and the like. See, e.g., the Merck Manual, 12th-17th Editions, Merck & Company, Rahway, NJ (1972, 1977, 1982, 1987, 1992, 1999), Pharmacotherapy Handbook, Wells et al., eds., Second Edition, Appleton and Lange, Stamford, Conn. (1998, 2000).

### Cardiovascular Disease

The present disclosure also provides a method for modulating or treating at least one cardiovascular disease in a cell, tissue, organ, animal, or patient, including, but not limited to, at least one of cardiac stun syndrome, myocardial infarction, congestive heart failure, stroke, ischemic stroke, hemorrhage, arteriosclerosis, atherosclerosis, restenosis, diabetic atherosclerotic disease, hypertension, arterial hypertension, renovascular hypertension, syncope, shock, syphilis of the cardiovascular system, heart failure, corpulmonale, primary pulmonary hypertension, cardiac arrhythmias, atrial ectopic beats, atrial flutter, atrial fibrillation (sustained or paroxysmal), post perfusion syndrome, cardiopulmonary bypass inflammation response, chaotic or multifocal atrial tachycardia, regular narrow QRS tachycardia, specific arrhythmias, ventricular fibrillation, His bundle arrhythmias, atrioventricular block, bundle branch block, myocardial ischemic disorders, coronary artery disease, angina pectoris, myocardial infarction, cardiomyopathy, dilated congestive cardiomyopathy, restrictive cardiomyopathy, valvular heart diseases, endocarditis, pericardial disease, cardiac tumors, aortic and peripheral aneurysms, aortic dissection, inflammation of the aorta, occlusion of the abdominal aorta and its branches, peripheral vascular disorders, occlusive arterial disorders, peripheral atherosclerotic disease, thromboangitis obliterans, functional peripheral arterial disorders, Raynoud's phenomenon and disease, acrocyanosis, erythromelalgia, venous diseases, venous thrombosis, varicose veins, arteriovenous fistula, lymphederma, lipedema, unstable angina, reperfusion injury, post pump syndrome, ischemia-reperfusion injury, and the like. Such a method can optionally comprise administering an effective amount of a composition or pharmaceutical composition comprising at least one anti-IL-17 antibody to a cell, tissue, organ, animal or patient in need of such modulation, treatment or therapy.

### Neurologic Disease

The present disclosure also provides a method for modulating or treating at least one neurologic disease in a cell, tissue, organ, animal or patient, including, but not limited to, at least one of: neurodegenerative diseases, multiple sclerosis, migraine headache, AIDS dementia complex, demyelinating diseases, such as multiple sclerosis and acute transverse myelitis; extrapyramidal and cerebellar disorders' such as lesions of the corticospinal system; disorders of the basal ganglia or cerebellar disorders; hyperkinetic movement disorders such as Huntington's Chorea and senile chorea; drug-induced movement disorders, such as those induced by drugs which block CNS dopamine receptors; hypokinetic movement disorders, such as Parkinson's disease; Progressive supranucleo Palsy; structural lesions of the cerebellum; spinocerebellar degenerations, such as spinal ataxia, Friedreich's ataxia, cerebellar cortical degenerations, multiple systems degenerations (Mencel, Dejerine-Thomas, Shi-Drager, and Machado-Joseph); systemic disorders (Refsum's disease, abetalipoprotemia, ataxia, telangictasia, and mitochondrial multi system disorder); demyelinating core disorders, such as multiple sclerosis, acute transverse myelitis; and disorders of the motor unit' such as neurogenic muscular atrophies (anterior horn cell degeneration, such as amyotrophic lateral sclerosis, infantile spinal muscular atrophy and juvenile spinal muscular atrophy); Alzheimer's disease; Down's Syndrome in middle age; Diffuse Lewy body disease; Senile Dementia of Lewy body type; Wernicke-Korsakoff syndrome; chronic alcoholism; Creutzfeldt-Jakob disease; sub acute sclerosing pan encephalitis, Hallerrorden-Spatz disease; and Dementia pugilistica, and the like. Such a method can optionally comprise administering an effective amount of a composition or pharmaceutical composition comprising at least one TNF antibody or specified portion or variant to a cell, tissue, organ, animal or patient in need of such modulation, treatment or therapy. See, e.g., the Merck Manual, 16th Edition, Merck & Company, Rahway, NJ (1992).

### Fibrotic Conditions

In addition to the above described conditions and diseases, the present disclosure also provides a method for modulating or treating fibrotic conditions of various etiologies such as liver fibrosis (including but not limited to alcohol-induced cirrhosis, viral-induced cirrhosis, autoimmune-induced hepatitis); lung fibrosis (including but not limited to scleroderma, idiopathic pulmonary fibrosis); kidney fibrosis (including but not limited to scleroderma, diabetic nephritis, glomerular nephritis, lupus nephritis); dermal fibrosis (including but not limited to scleroderma, hypertrophic and keloid scarring, burns); myelofibrosis; Neurofibromatosis; fibroma; intestinal fibrosis; and fibrotic adhesions resulting from surgical procedures.

In such a method, the fibrosis can be organ specific fibrosis or systemic fibrosis. The organ specific fibrosis can be associated with at least one of lung fibrosis, liver fibrosis, kidney fibrosis, heart fibrosis, vascular fibrosis, skin fibrosis, eye fibrosis, bone marrow fibrosis or other fibrosis. The lung fibrosis can be associated with at least one of idiopathic pulmonary fibrosis, drug induced pulmonary fibrosis, asthma, sarcoidosis or chronic obstructive pulmonary disease. The liver fibrosis can be associated with at least one of cirrhosis, schistomasomiasis or cholangitis. The cirrhosis can be selected from alcoholic cirrhosis, post-hepatitis C cirrhosis, primary biliary cirrhosis. The cholangitis is sclerosing cholangitis. The kidney fibrosis can be associated with at least one of diabetic nephropathy, IgA nephropathy, transplant nephropathy, or lupus nephritis. The heart fibrosis can be associated with at least one type of myocardial infarction. The vascular fibrosis can be associated with at least one of postangioplasty arterial restenosis, or atherosclerosis. The skin fibrosis can be associated with at least one of burn scarring, hypertrophic scarring, keloid, or nephrogenic fibrosing dermatopathy. The eye fibrosis can be associated with at least one of retro-orbital fibrosis, postcataract surgery or proliferative vitreoretinopathy. The the bone marrow fibrosis can be associated with at least one of idiopathic myelofibrosis or drug induced myelofibrosis. The other fibrosis can be selected from Peyronie's disease, Dupuytren's contracture or dermatomyositis. The systemic fibrosis can be selected from systemic sclerosis and graft versus host disease.

The present disclosure also provides a method for modulating or treating at least one wound, trauma or tissue injury or chronic condition resulting from or related thereto, in a cell, tissue, organ, animal or patient, including, but not limited to, at least one of: bodily injury or a trauma associated with surgery including thoracic, abdominal, cranial, or oral surgery; or wherein the wound can be selected from the group consisting of aseptic wounds, contused wounds, incised wounds, lacerated wounds, non-penetrating wounds, open wounds, penetrating wounds, perforating wounds, puncture wounds, septic wounds, infarctions and subcutaneous wounds; or wherein the wound can be selected from the group consisting of ischemic ulcers, pressure sores, fistulae, severe bites, thermal burns and donor site wounds; or wherein the wound is an aphthous wound, a traumatic wound or a herpes associated wound. Donor site wounds are wounds which e.g. occur in connection with removal of hard tissue from one part of the body to another part of the body e.g. in connection with transplantation. The wounds resulting from such operations are very painful and an improved healing is therefore most valuable. Wound fibrosis is also amenable to anti-IL-17 antibody therapy as the first cells to invade the wound area are neutrophils followed by monocytes, which are activated by macrophages. Macrophages are believed to be essential for efficient wound healing in that they also are responsible for phagocytosis of pathogenic organisms and a clearing up of tissue debris. Furthermore, they release numerous factors involved in subsequent events of the healing process. The macrophages attract fibroblasts, which start the production of collagen. Almost all tissue repair processes include the early connective tissue formation, a stimulation of this and the subsequent processes improve tissue healing, however, overproduction of connective tissue and collegen can lead to a fibrotic tissue characterized as inelastic and hypoxic. The anti-IL-17 antibodies of the invention can be used in methods for modulating, treating or preventing such sequelae of wound healing.

The present antibodies of the present invention may also be used in methods for modulating or treating at least one symptom of chronic rejection of a transplanted organ, tissue or cell, such as a cardiac transplant.

### Other Therapeutic Uses of Anti-IL-17 Antibodies

The present disclosure also provides a method for modulating or treating at least one infectious disease in a cell, tissue, organ, animal or patient, including, but not limited to, at least one of: acute or chronic bacterial infection, acute and chronic parasitic or infectious processes, including bacterial, viral and fungal infections, HIV infection/HIV neuropathy, meningitis, hepatitis (A, B or C, or the like), septic arthritis, peritonitis, pneumonia, epiglottitis, e. coli 0157:h7, hemolytic uremic syndrome/thrombolytic thrombocytopenic purpura, malaria, dengue hemorrhagic fever, leishmaniasis, leprosy, toxic shock syndrome, streptococcal myositis, gas gangrene, mycobacterium tuberculosis, mycobacterium avium intracellulare, pneumocystis carinii pneumonia, pelvic inflammatory disease, orchitis/epidydimitis, legionella, lyme disease, influenza a, epstein-barr virus, vital-associated hemaphagocytic syndrome, vital encephalitis/aseptic meningitis, and the like.

Any method of the present disclosure can comprise administering an effective amount of a composition or pharmaceutical composition comprising at least one anti-IL-17 antibody to a cell, tissue, organ, animal or patient in need of such modulation, treatment or therapy. Such a method can optionally further at least one selected from at least one TNF antagonist (e.g., but not limited to a TNF antibody or fragment, a soluble TNF receptor or fragment, fusion proteins thereof, or a small molecule TNF antagonist), an antirheumatic (e.g., methotrexate, auranofin, aurothioglucose, azathioprine, etanercept, gold sodium thiomalate, hydroxychloroquine sulfate, leflunomide, sulfasalzine), a muscle relaxant, a narcotic, a non-steroid anti-inflammatory drug (NSAID), an analgesic, an anesthetic, a sedative, a local anethetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod (dexamethasone), an anabolic steroid (testosterone), a diabetes related agent, a mineral, a nutritional, a thyroid agent, a vitamin, a calcium related hormone, an antidiarrheal, an antitussive, an antiemetic, an antiulcer, a laxative, an anticoagulant, an erythropoietin (e.g., epoetin alpha), a filgrastim (e.g., G-CSF, Neupogen), a sargramostim (GM-CSF, Leukine), an immunization, an immunoglobulin (rituximab), an immunosuppressive (e.g., basiliximab, cyclosporine, daclizumab), a growth hormone, a hormone antagonist, a reproductive hormone antagonist (flutamide, nilutamide), a hormone release modulator (leuprolide, goserelin), a hormone replacement drug, an estrogen receptor modulator (tamoxifen), a retinoid (tretinoin), a topoisomerase inhibitor (etoposide, irinotecan), a cytoxin (doxorubicin), a mydriatic, a cycloplegic, an alkylating agent (carboplatin), a nitrogen mustard (melphalen, chlorabucil), a nitrosourea (carmustine, estramustine) an antimetabolite (methotrexate, cytarabine, fluorouracil), a mitotic inhibitor (vincristine, taxol), a radiopharmaceutical (Iodine131-tositumomab), a radiosensitizer (misonidazole, tirapazamine) an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, dornase alpha (Pulmozyme), a cytokine (interferon alpha-2, IL2) or a cytokine antagonist (inflixamab).. Suitable dosages are well known in the art. See, e.g., Wells et al., eds., Pharmacotherapy Handbook, 2nd Edition, Appleton and Lange, Stamford, CT (2000); PDR Pharmacopoeia, Tarascon Pocket Pharmacopoeia 2000, Deluxe Edition, Tarascon Publishing, Loma Linda, CA (2000).

Particular combinations for treatment of neoplastic diseases comprise co-administration or combination therapy by administering, before concurrently, and/or after, an antineplastic agent such as an alkylating agent, a nitrogen mustard, a nitrosurea, an antibiotic, an anti-metabolite, a hormonal agonist or antagonist, an immunomodulator, and the like. For use in metastatic melanoma and other neoplastic diseases, a preferred combination is to co-administer the antibody with dacarbazine, interferon alpha, interleukin-2, temozolomide, cisplatin, vinblastine, Imatinib Mesylate, carmustine, paclitaxel and the like. For metastatic melanoma, dacarbazine is preferred.

### 11. Dosages and Methods of Administration

A method of the present invention can comprise a method for treating a IL-17 mediated disorder, comprising administering an effective amount of a composition or pharmaceutical composition comprising at least one anti-IL-17 antibody to a cell, tissue, organ, animal or patient in need of such modulation, treatment or therapy. Such a method can optionally further comprise co-administration or combination therapy for treating such diseases or discorders, wherein the administering of said at least one anti-IL-17 antibody, specified portion or variant thereof, further comprises administering, before concurrently, and/or after, at least one selected from a renal drug, a dermatogical drug, an anti-angiogenic drug, an anti-infective drug, a cardiovascular (CV) system drug, a central nervous system (CNS) drug, an autonomic nervous system (ANS) drug, a respiratory tract drug, a gastrointestinal (GI) tract drug, a hormonal drug, a drug for fluid or electrolyte balance, a hematologic drug, an antineoplactic, an immunomodulation drug, an ophthalmic, otic or nasal drug, a topical drug, a nutritional drug or the like, at least one TNF antagonist (e.g., but not limited to a TNF antibody or fragment, a soluble TNF receptor or fragment, fusion proteins thereof, or a small molecule TNF antagonist), an antirheumatic (e.g., methotrexate, auranofin, aurothioglucose, azathioprine, etanercept, gold sodium thiomalate, hydroxychloroquine sulfate, leflunomide, sulfasalzine), a muscle relaxant, a narcotic, a non-steroid anti-inflammatory drug (NSAID), an analgesic, an anesthetic, a sedative, a local anethetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod, an anabolic steroid, a diabetes related agent, a mineral, a nutritional, a thyroid agent, a vitamin, a calcium related hormone, an antidiarrheal, an antitussive, an antiemetic, an antiulcer, a laxative, an anticoagulant, an erythropieitin (e.g., epoetin alpha), a filgrastim (e.g., G-CSF, Neupogen), a sargramostim (GM-CSF, Leukine), an immunization, an immunoglobulin, an immunosuppressive (e.g., basiliximab, cyclosporine, daclizumab), a growth hormone, a hormone replacement drug, an estrogen receptor modulator, a mydriatic, a cycloplegic, an alkylating agent, an antimetabolite, a mitotic inhibitor, a radiopharmaceutical, an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, dornase alpha (Pulmozyme), a cytokine or a cytokine antagonist. Such drugs are well known in the art, including formulations, indications, dosing and administration for each presented herein (see., e.g., Nursing 2001 Handbook of Drugs, 21st edition, Springhouse Corp., Springhouse, PA, 2001; Health Professional's Drug Guide 2001, ed., Shannon, Wilson, Stang, Prentice-Hall, Inc, Upper Saddle River, NJ; Pharmcotherapy Handbook, Wells et al., ed., Appleton & Lange, Stamford, CT).

Typically, treatment of pathologic conditions is effected by administering an effective amount or dosage of at least one anti-IL-17 antibody composition that total, on average, a range from at least about 0.01 to 500 milligrams of at least one anti-IL-17antibody per kilogram of patient per dose, and preferably from at least about 0.1 to 100 milligrams antibody /kilogram of patient per single or multiple administration, depending upon the specific activity of contained in the composition. Alternatively, the effective serum concentration can comprise 0.1-5000 µg/ml serum concentration per single or multiple adminstration. Suitable dosages are known to medical practitioners and will, of course, depend upon the particular disease state, specific activity of the composition being administered, and the particular patient undergoing treatment. In some instances, to achieve the desired therapeutic amount, it can be necessary to provide for repeated administration, *i.e.,* repeated individual administrations of a particular monitored or metered dose, where the individual administrations are repeated until the desired daily dose or effect is achieved.

Preferred doses can optionally include 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and/or 100-500 mg/kg/administration, or any range, value or fraction thereof, or to achieve a serum concentration of 0.1, 0.5, 0.9, 1.0, 1.1, 1.2, 1.5, 1.9, 2.0, 2.5,2.9, 3.0, 3.5, 3.9,4.0,4.5,4.9, 5.0, 5.5, 5.9, 6.0, 6.5, 6.9, 7.0, 7.5, 7.9, 8.0, 8.5, 8.9, 9.0, 9.5, 9.9, 10, 10.5, 10.9, 11, 11.5, 11.9, 20, 12.5, 12.9, 13.0, 13.5, 13.9, 14.0, 14.5, 4.9, 5.0, 5.5., 5.9, 6.0, 6.5, 6.9, 7.0, 7.5, 7.9, 8.0, 8.5, 8.9, 9.0, 9.5, 9.9, 10, 10.5, 10.9, 11, 11.5, 11.9, 12, 12.5, 12.9, 13.0, 13.5, 13.9, 14, 14.5, 15, 15.5, 15.9, 16, 16.5, 16.9, 17, 17.5, 17.9, 18, 18.5, 18.9, 19, 19.5, 19.9, 20, 20.5, 20.9, 21, 22, 23, 24, 25, 26, 27, 28, 29, 30, 35, 40, 45, 50, 55, 60, 65, 70, 75, 80, 85, 90, 96, 100, 200, 300, 400, 500, 600, 700, 800, 900, 1000, 1500, 2000, 2500, 3000, 3500, 4000, 4500, and/or 5000 µg/ml serum concentration per single or multiple administration, or any range, value or fraction thereof.

Alternatively, the dosage administered can vary depending upon known factors, such as the pharmacodynamic characteristics of the particular agent, and its mode and route of administration; age, health, and weight of the recipient; nature and extent of symptoms, kind of concurrent treatment, frequency of treatment, and the effect desired. Usually a dosage of active ingredient can be about 0.1 to 100 milligrams per kilogram of body weight. Ordinarily 0.1 to 50, and preferably 0.1 to 10 milligrams per kilogram per administration or in sustained release form is effective to obtain desired results.

As a non-limiting example, treatment of humans or animals can be provided as a one-time or periodic dosage of at least one antibody of the present invention 0.1 to 100 mg/kg, such as 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively or additionally, at least one of week 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52, or alternatively or additionally, at least one of 1, 2, 3, 4, 5, 6,, 7, 8, 9, 10, 11, 12, 13, 14, 15, 16, 17, 18, 19, or 20 years, or any combination thereof, using single, infusion or repeated doses.

Dosage forms (composition) suitable for internal administration generally contain from about 0.001 milligram to about 500 milligrams of active ingredient per unit or container. In these pharmaceutical compositions the active ingredient will ordinarily be present in an amount of about 0.5-99.999% by weight based on the total weight of the composition.

For parenteral administration, the antibody can be formulated as a solution, suspension, emulsion, particle, powder, or lyophilized powder in association, or separately provided, with a pharmaceutically acceptable parenteral vehicle. Examples of such vehicles are water, saline, Ringer's solution, dextrose solution, and 1-10% human serum albumin. Liposomes and nonaqueous vehicles such as fixed oils can also be used. The vehicle or lyophilized powder can contain additives that maintain isotonicity (e.g., sodium chloride, mannitol) and chemical stability (e.g., buffers and preservatives). The formulation is sterilized by known or suitable techniques. Suitable pharmaceutical carriers are described in the most recent edition of Remington's Pharmaceutical Sciences, A. Osol, a standard reference text in this field. **Alternative Administration.** Many known and developed modes of can be used according to the present invention for administering pharmaceutically effective amounts of at least one anti-IL-17 antibody according to the present invention. While pulmonary administration is used in the following description, other modes of administration can be used according to the present invention with suitable results. IL-17 antibodies of the present invention can be delivered in a carrier, as a solution, emulsion, colloid, or suspension, or as a dry powder, using any of a variety of devices and methods suitable for administration by inhalation or other modes described here within or known in the art.

**Parenteral Formulations and Administration.** Formulations for parenteral administration can contain as common excipients sterile water or saline, polyalkylene glycols such as polyethylene glycol, oils of vegetable origin, hydrogenated naphthalenes and the like. Aqueous or oily suspensions for injection can be prepared by using an appropriate emulsifier or humidifier and a suspending agent, according to known methods. Agents for injection can be a non-toxic, non-orally administrable diluting agent such as aquous solution or a sterile injectable solution or suspension in a solvent. As the usable vehicle or solvent, water, Ringer's solution, isotonic saline, etc. are allowed; as an ordinary solvent, or suspending solvent, sterile involatile oil can be used. For these purposes, any kind of involatile oil and fatty acid can be used, including natural or synthetic or semisynthetic fatty oils or fatty acids; natural or synthetic or semisynthtetic mono- or di- or tri-glycerides. Parental administration is known in the art and includes, but is not limited to, conventional means of injections, a gas pressured needle-less injection device, or laser perforator devise, as well known in the art (e.g., but not limited to, materials and methods disclosed in U.S. Pat. No. 5,851,198, and U.S. Pat. No. 5,839,446).

**Alternative Delivery.** The invention further relates to the administration of at least one anti-IL-17 antibody by parenteral, subcutaneous, intramuscular, intravenous, intrarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal means. At least one anti-IL-17 antibody composition can be prepared for use for parenteral (subcutaneous, intramuscular or intravenous) or any other administration particularly in the form of liquid solutions or suspensions; for use in vaginal or rectal administration particularly in semisolid forms such as, but not limited to, creams and suppositories; for buccal, or sublingual administration such as, but not limited to, in the form of tablets or capsules; or intranasally such as, but not limited to, the form of powders, nasal drops or aerosols or certain agents; or transdermally such as not limited to a gel, ointment, lotion, suspension or patch delivery system with chemical enhancers such as dimethyl sulfoxide to either modify the skin structure or to increase the drug concentration in the transdermal patch (Junginger, et al. In "Drug Permeation Enhancement"; Hsieh, D. S., Eds., pp. 59-90 (Marcel Dekker, Inc. New York 1994, entirely incorporated herein by reference), or with oxidizing agents that enable the application of formulations containing proteins and peptides onto the skin (WO 98/53847), or applications of electric fields to create transient transport pathways such as electroporation, or to increase the mobility of charged drugs through the skin such as iontophoresis, or application of ultrasound such as sonophoresis (U.S. Pat. Nos. 4,309,989 and 4,767,402).

**Pulmonary/Nasal Administration.** For pulmonary administration, preferably at least one anti-IL-17 antibody composition is delivered in a particle size effective for reaching the lower airways of the lung or sinuses. According to the invention, at least one anti-IL-17 antibody can be delivered by any of a variety of inhalation or nasal devices known in the art for administration of a therapeutic agent by inhalation. These devices capable of depositing aerosolized formulations in the sinus cavity or alveoli of a patient include metered dose inhalers, nebulizers, dry powder generators, sprayers, and the like. Other devices suitable for directing the pulmonary or nasal administration of antibodies are also known in the art. All such devices can use of formulations suitable for the administration for the dispensing of antibody in an aerosol. Such aerosols can be comprised of either solutions (both aqueous and non aqueous) or solid particles. Metered dose inhalers like the Ventolin^{®} metered dose inhaler, typically use a propellent gas and require actuation during inspiration (See, e.g., WO 94/16970, WO 98/35888). Dry powder inhalers like Turbuhaler™ (Astra), Rotahaler^{®}

(Glaxo), Diskus^{®} (Glaxo), Spiros™ inhaler (Dura), devices marketed by Inhale Therapeutics, and the Spinhaler^{®} powder inhaler (Fisons), use breath-actuation of a mixed powder (US 4668218 Astra, EP 237507 Astra, WO 97/25086 Glaxo, WO 94/08552 Dura, US 5458135 Inhale, WO 94/06498 Fisons, entirely incorporated herein by reference). Nebulizers like AERx™ Aradigm, the Ultravent^{®} nebulizer (Mallinckrodt), and the Acorn II^{®} nebulizer (Marquest Medical Products) (US 5404871 Aradigm, WO 97/22376), the above references entirely incorporated herein by reference, produce aerosols from solutions, while metered dose inhalers, dry powder inhalers, etc. generate small particle aerosols. These specific examples of commercially available inhalation devices are intended to be a representative of specific devices suitable for the practice of this invention, and are not intended as limiting the scope of the invention. Preferably, a composition comprising at least one anti-IL-17 antibody is delivered by a dry powder inhaler or a sprayer. There are a several desirable features of an inhalation device for administering at least one antibody of the present invention. For example, delivery by the inhalation device is advantageously reliable, reproducible, and accurate. The inhalation device can optionally deliver small dry particles, e.g. less than about 10 µm, preferably about 1-5 µm, for good respirability.

### EXAMPLE 1: IL-17 Stimulates Pro-Fibrotic Gene Expression

### Methods:

Human renal proximal tubule epithelial cells (HK-2) (ATCC, Manassas, VA) were grown in Keratinocyte Serum-free Medium (Invitrogen/Gibco, Carlsbad, CA) containing recombinant human epidermal growth factor (5 ng/ml) and bovine pituitary extract (0.05 mg/ml). Cells were plated in 24-well plates at 25,000 cells per well and allowed to adhere for 24 h. Cell growth medium was then replaced with supplement-free medium and cells were incubated overnight. Cells were then stimulated for 24 h in the presence or absence of recombinant human IL-17 (R&D Systems, Minneapolis, MN) at 10 or 100 ng/ml. RNA was subsequently isolated using the RNeasy Plus Mini-Kit (Qiagen, Valencia, CA) and reverse transcribed into cDNA using TaqMan@ Reverse Transcription Reagents (Applied Biosystems, Foster City, CA). Pro-fibrotic gene expression was determined by real-time PCR analysis using Taqman® Universal PCR Master Mix (Applied Biosystems) and Taqman® Gene Expression Assays (Applied Biosystems).

Quantitative gene expression was calculated by the comparative C_{T} method, where C_{T} values are determined as the threshold cycle number for which gene expression is first detected. For the genes of interest, fold changes in gene expression were first normalized to the housekeeping gene 18S, giving ΔΔC_{T} values. Fold changes in gene expression due to IL-17 treatment were calculated by ΔΔC_{T} = ΔC_{T(unstimulated)} -ΔC_{T(stimulated)}, where the unstimulated sample served as calibrator. Statistically significant differences were determined by the Student t-test. (See Figure 1)

### IL-17 Stimulates Secretion of Pro-Fibrotic Soluble Mediators

### Methods:

Human proximal tubule epithelial cells were grown in the same medium as described above. Cells were plated in 24-well plates at 25,000 cells per well and allowed to adhere for 24 h. Cell growth medium was then replaced with supplement-free medium and cells were incubated overnight. Cells were then stimulated for 48 h in the presence or absence of recombinant human IL-17 (R&D Systems, Minneapolis, MN) at 1, 10 or 100 ng/ml. For antibody neutralization, cells were plated in 6-well plates at 100,000 cells per well. IL-17 (10 ng/ml) was pre-incubated with mouse monoclonal anti-human IL-17 antibody (120 ng/ml)(eBioscience, San Diego, CA) for 1 h, RT prior to applying to cells.

Cytokine profiling was determined using a human cytokine/chemokine premixed *Lincoplex* kit (MCYTO-60K-PMX30; Linco Research, Millipore; St. Charles, Missouri), containing antibody-immobilized beads for the following human analytes: IL-1β, IL-2, IL-1Rα, IL-4, IL-5, EGF, IL-6, IL-7, TGFα, Fractalkine, IL-8, IL-10, IL-12(p40), IL-12(p70), TNF-α, IFN-y, GM-CSF, MIP-1α, MIP-1β, MCP-1, RANTES, IL-13, IL-lα, IL-15, IL-17, IP-10, Eotaxin, sCD40L, VEGF, and G-CSF. The immunoassay was performed according to manufacturer's instructions. Briefly, frozen serum samples were thawed and standards were diluted in cell culture media. Following completion of the immunoassay, the samples were run on a Bio-Plex™ (Bio-Rad Laboratories, Inc; Hercules, CA). A five-parameter logistic regression algorithm was used to determine the analyte level in the supernatant based on a 6-point standard curve. Statistically significant differences were determined by the Student t-test. (See Figures 2 and 3)

### IL-17 Downregulates ZO-1 Protein Expression

### Methods:

Human proximal tubule epithelial cells were grown in the same medium as described above. Cells were plated in 6-well plates on sterile coverslips (18 x 18 mm) at 125,000 cells per well and allowed to adhere for 24 h. Cell growth medium was then replaced with supplement-free medium and cells were incubated overnight. Cells were then stimulated for 48 h in the presence or absence of 50 ng/ml recombinant human IL-17 (R&D Systems, Minneapolis, MN). The antibody neutralization experiment was performed as described above except with 0.6 µg/ml mouse monoclonal anti-human IL-17 antibody (eBioscience, San Diego, CA). Cells were fixed in 4% paraformaldehyde (15 min, RT), permeabilized with 0.5% Triton X-100 (8 min, RT) and blocked with Powerblock (Biogenex, San Ramon, CA) for 8 min, RT. ZO-1 was detected utilizing monoclonal mouse anti-human ZO-1 (18 µg/ml) (Invitrogen, Carlsbad, CA) primary antibody in combination with donkey anti-mouse Alexa Fluor 594 (8 µg/ml) (Invitrogen, Carlsbad, CA) secondary antibody. Coverslips were overturned on a microscope slide containing - 8 µl of SlowFade Gold Antifade reagent (Invitrogen, Carlsbad, CA). Slides were visualized on a Zeiss axiophot microscope. (See Figure 4)

### IV. Advantages

As discussed in Section I, previous studies have demonstrated the capacity for IL-17 to stimulate the secretion of pro-inflammatory mediators from renal proximal tubule epithelium. The present study expands on these findings and provides novel results by demonstrating IL-17-induced secretion of another known inflammatory factor (VEGF) as well as IL-17-induced secretion of molecules known to recruit immune cells and mediate their direct interaction with resident epithelial cells (G-CSF, GM-CSF and fractalkine). The present study also demonstrates, for the first time, direct action of IL-17 on the tubular epithelium to induce a pro-fibrotic phenotypic transition. IL-17 induced the downregulation of the pro-epithelial e-cadherin gene (CDH1) and upregulation of genes associated with matrix deposition and myofibroblast differentiation (CTGF) and immune cell interaction and activation (CD44). Also, a direct effect on epithelial integrity was demonstrated by IL-17-induced ZO-1 downregulation. The direct pro-fibrotic action in combination with stimulating the secretion of soluble pro-inflammatory/fibrotic mediators indicates IL-17 is a potent renal inflammatory/fibrogenic molecule. These new findings, in combination with the previously described pro-inflammatory activities of IL-17, provide rationale for the neutralization of IL-17 as a novel anti-inflammatory/fibrotic therapeutic approach for the attenuation of renal inflammatory/fibrotic disorders and potentially other pathologies manifesting the same pathogenesis.

### References

1. Freese P, Svalander CT, Molne J, Norden G and Nyberg G. Chronic allograft nephropathy-biopsy findings and outcome. Nephrol Dial Transplant 2001; 16: 2401-2406.
2. Ritz E, Keller C and Bergis KH. Nephropathy of type II diabetes mellitus. Nephrol Dial Transplant 1996; 11 Suppl 9: 38-44. Review.
3. Liu Y. Renal fibrosis: new insights into the pathogenesis and therapeutics. Kidney Int 2006; 69: 213-217. Review.
4. Simonson MS. Phenotypic transitions and fibrosis in diabetic nephropathy. Kidney Int 2007; 71: 846-854.
5. Phillips AO and Steadman R. Diabetic Nephropathy: The central role of renal proximal tubular cells in tubulointerstitial injury. Histol Histopathol 2002; 17: 247-52. Review.
6. Phillips AO. The Role of Proximal Tubular Cells in Interstitial Fibrosis: Understanding TGF-b1. Chang Gung Med J 2007; 30: 2-6. Review.
7. Van Kooten C, Boonstra JG, Paape M, Fossiez F, Banchereau J, Lebecque S, Bruijn JA, De Fijter JW, Van Es LA and Daha M. Interleukin-17 activates human renal epithelial cells in vitro and is expressed during renal allograft rejection. J Am Soc Nephrol 1998; 9: 1526-34.
8. Loong C-C, Hsieh H-G, Lui W-Y, Chen A and Lin C-Y. Evidence for the early involvement of interleukin 17 in human and experimental renal allograft rejection. J Path 2002; 197: 322-32.
9. Woltman AM, De Haij S, Boonstra JG, Gobin SJP, Daha MR and Van Kooten C. Interleukin-17 and CD40-Ligand synergistically enhance cytokine and chemokine production by renal epithelial cells. J Am Soc Nephrol 2000; 11: 2044-55.
10. Racusen LC, Solez K, Colvin RB, et al. The Banff 97 working classification of renal allograft pathology. Kidney Int 1999; 55: 713-23.
11. Mannon RB. Therapeutic targets in the treatment of allograft fibrosis. Am J Transpl 2006; 6: 867-75.
12. Thomas MC, Burns WC and Cooper ME. Tubular changes in early diabetic nephropathy. Adv Chron Kid Dis 2005; 12(2):177-86.
13. Eitner F and Floege J. Therapeutic targets for prevention and regression of progressive fibrosing renal diseases. Curr Opin Invistig Drugs 2005; 6(3): 255-61.
14. Florquin S and Rouschop KM. Reciprocal functions of hepatocyte growth factor and transforming growth factor-beta 1 in the progression of renal diseases: a role for CD44? Kid Int Suppl 2003 Oct; (86): S15-20.
15. Stievano L, Piovan E and Amadori A. The biology of human granulocyte-macrophage colony-stimulating factor (GM-CSF). Crit Rev Immunol 2004; 24(3): 205-28.
16. Eyles JL, Roberts AW, Metcalf D and Wicks IP. Granulocyte colony-stimulating factor and neutrophils--forgotten mediators of inflammatory disease. Nat Clin Pract Rheumatol 2006 Sep; 2(9): 500-10.

## Claims

1. An interleukin-17 (IL-17) antagonist for use in treating kidney or renal fibrosis, wherein said IL-17 is selected from IL17A or IL-17F, wherein said antagonist is an antibody.

2. The IL-17 antagonist for use according to claim 1, wherein said antibody comprises at least one variable region comprising at least one heavy chain variable region and at least one light chain, and said IL-17 antibody binds at least one of SEQ ID NOS: 1-4.

3. The IL-17 antagonist for use according to claim 1, wherein said antibody binds IL-17 with an affinity of at least one selected from at least 10⁻⁹ M, at least 10⁻¹⁰ M, at least 10⁻¹¹ M, or at least 10⁻¹² M.

4. The IL-17 antagonist for use according to claim 1, wherein said antibody substantially modulates at least one activity of at least one IL-17 polypeptide.

5. The IL-17 antagonist for use according to claim 1, wherein said antibody is provided as a composition further comprising at least one pharmaceutically acceptable carrier or diluent.

6. The IL-17 antagonist for use according to claim 1, which is suitable for administering with at least one compound or polypeptide selected from at least one of a detectable label or reporter, a TNF antagonist, an anti-infective drug, a cardiovascular (CV) system drug, a central nervous system (CNS) drug, an autonomic nervous system (ANS) drug, a respiratory tract drug, a gastrointestinal (GI) tract drug, a hormonal drug, a drug for fluid or electrolyte balance, a hematologic drug, an antineoplastic, an immunomodulation drug, an opthalmic, otic or nasal drug, a topical drug, a nutritional product, a cytokine, or a cytokine antagonist.

7. The IL-17 antagonist for use according to claim 1, wherein said inhibiting effective amount is 0.001-50 mg/kilogram of said cells, tissue, organ or animal.

8. The IL-17 antagonist for use according to claim 1, which is suitable for administering by at least one mode selected from parenteral, subcutaneous, intramuscular, intravenous, intrarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal.

9. The IL-17 antagonist for use according to claim 1, wherein said kidney fibrosis is associated with lupus glomeruloschelerosis.

## Patentansprüche

1. Interleukin-17(IL-17)-Antagonist zur Verwendung in der Behandlung von Nierenfibrose (renaler Fibrose), wobei das IL-17 aus IL-17A oder IL-17F ausgewählt ist, wobei es sich bei dem Antagonisten um einen Antikörper handelt.

2. IL-17-Antagonist zur Verwendung nach Anspruch 1, wobei der Antikörper mindestens eine variable Region umfasst, die mindestens eine variable Region der schweren Kette und mindestens eine leichte Kette umfasst, und der IL-17-Antikörper an mindestens eine der SEQ. ID. NO: 1-4 bindet.

3. IL-17-Antagonist zur Verwendung nach Anspruch 1, wobei der Antikörper IL-17 mit einer Affinität von mindestens einer ausgewählt aus mindestens 10⁻⁹ M, mindestens 10⁻¹⁰ M, mindestens 10⁻¹¹ M oder mindestens 10⁻¹² m ausgewählt ist.

4. IL-17-Antagonist zur Verwendung nach Anspruch 1, wobei der Antikörper im Wesentlichen zumindest eine Aktivität von mindestens einem IL-17-Polypeptid moduliert.

5. IL-17-Antagonist zur Verwendung nach Anspruch 1, wobei der Antikörper als Zusammensetzung bereitgestellt wird, die weiterhin mindestens einen pharmazeutisch unbedenklichen Träger oder mindestens ein pharmazeutisch unbedenkliches Verdünnungsmittel umfasst.

6. IL-17-Antagonist zur Verwendung nach Anspruch 1, der sich zur Verabreichung mit mindestens einer Verbindung oder mindestens einem Polypeptid, ausgewählt aus mindestens einem der folgenden: nachweisbare Markierung oder nachweisbarer Reporter, TNF-Antagonist, Anti-Infektions-Arzneistoff, Herz-Kreislauf-System-Arzneistoff, Zentralnervensystem-Arzneistoff, ANS (Autonomes Nervensystem)-Arzneistoff, Atemwegsarzneistoff, Magen-Darm-Arzneistoff, Hormonarzneistoff, Arzneistoff für den Flüssigkeits-oder Elektrolytenhaushalt, hämatologischer Arzneistoff, Antineoplastikum, Immunmodulationsarzneistoff, ophthalmischer, otischer oder nasaler Arzneistoff, topischer Arzneistoff, Nährmittelprodukt, Cytokin oder Cytokinantagonist ausgewählt ist.

7. IL-17-Antagonist zur Verwendung nach Anspruch 1, wobei die inhibitionswirksame Menge 0,001-50 mg/Kilogramm dieser Zellen, dieses Gewebes, Organs oder Tiers beträgt.

8. IL-17-Antagonist zur Verwendung nach Anspruch 1, der sich für die Verabreichung nach mindestens einem Modus, ausgewählt aus parenteral, subkutan, intramuskulär, intravenös, intraartikulär, intrabronchial, intraabdominal, intrakapsulär, intrakartilaginär, intrakavitär, intrazölial, intrazerebellar, intrazerebroventrikulär, intrakolisch, intrazervical, intragastrisch, intrahepatisch, intramyokardial, intraoseal, intrapelvin, intraperikardial, intraperitoneal, intrapleural, intraprostatisch, intrapulmonal, intrarektal, intrarenal, intraretinal, intraspinal, intrasynovial, intrathorakal, intrauterin, intravesikal, intraläsional, als Bolus, vaginal, rektal, bukkal, sublingual, intranasal und transdermal, geeignet ist.

9. IL-17-Antagonist zur Verwendung nach Anspruch 1, wobei die Nierenfibrose mit Lupus-Glomerulosklerose assoziiert ist.

## Revendications

1. Antagoniste d'interleukine 17 (IL-17) pour utilisation dans le traitement de la fibrose du rein ou rénale, ledit IL-17 étant choisi parmi IL17A ou IL-17F, ledit antagoniste étant un anticorps.

2. Antagoniste d'IL-17 pour utilisation selon la revendication 1, ledit anticorps comprenant au moins une région variable comprenant au moins une région variable de chaîne lourde et au moins une chaîne légère, et ledit anticorps contre IL-17 se liant à au moins l'un de SEQ ID NO: 1-4.

3. Antagoniste d'IL-17 pour utilisation selon la revendication 1, ledit anticorps se liant à IL-17 avec une affinité d'au moins l'un choisi parmi au moins 10⁻⁹ M, au moins 10⁻¹⁰ M, au moins 10⁻¹¹ M, ou au moins 10⁻¹² M.

4. Antagoniste d'IL-17 pour utilisation selon la revendication 1, ledit anticorps modulant sensiblement au moins une activité d'au moins un polypeptide IL-17.

5. Antagoniste d'IL-17 pour utilisation selon la revendication 1, ledit anticorps étant fourni sous la forme d'une composition comprenant en outre au moins un véhicule ou diluant pharmaceutiquement acceptable.

6. Antagoniste d'IL-17 pour utilisation selon la revendication 1, qui est adapté pour administration avec au moins un composé ou polypeptide choisi parmi au moins l'un d'un marqueur ou rapporteur détectable, un antagoniste de TNF, un médicament anti-infectieux, un médicament du système cardiovasculaire (CV), un médicament du système nerveux central (SNC), un médicament du système nerveux autonome (SNA), un médicament du tractus respiratoire, un médicament du tractus gastro-intestinal (GI), un médicament hormonal, un médicament pour l'équilibre des fluides ou des électrolytes, un médicament hématologique, un antinéoplasique, un médicament immunomodulateur, un médicament ophtalmique, otique ou nasal, un médicament topique, un produit nutritionnel, une cytokine ou un antagoniste de cytokine.

7. Antagoniste d'IL-17 pour utilisation selon la revendication 1, ladite quantité efficace inhibitrice étant de 0,001 à 50 mg/kilogramme desdits cellules, tissu, organe ou animal.

8. Antagoniste d'IL-17 pour utilisation selon la revendication 1, qui est adapté pour administration par au moins un mode choisi parmi les modes parentéral, sous-cutané, intramusculaire, intraveineux, intra-articulaire, intrabronchique, intra-abdominal, intracapsulaire, intracartilagineux, endocavitaire, intracoeliaque, intracérébelleux, intracérébroventriculaire, intracolique, intracervical, intragastrique, intrahépatique, intramyocardique, intra-osseux, intrapelvien, intrapéricardique, intrapéritonéal, intrapleural, intraprostatique, intrapulmonaire, intrarectal, intrarénal, intrarétinien, intraspinal, intrasynovial, intrathoracique, intra-utérin, intravésical, intralésionnel, en bolus, vaginal, rectal, buccal, sublingual, intranasal ou transdermique.

9. Antagoniste d'IL-17 pour utilisation selon la revendication 1, ladite fibrose du rein étant associée à la glomérulosclérose liée au lupus.


### SEQUENCE LISTING

<110> Dudas, Paul; Elloso, M. Merle; Sague, Sarah; Farrell, Francis
<120> METHODS AND COMPOSITIONS FOR TREATING FIBROSIS RELATED DISORDERS USING IL-17 ANTAGONISTS
<130> CEN5190WOPCT
<150> 60/
   <151>
<160> 4
<170> PatentIn version 3.3
<210> 1
   <211> 155
   <212> PRT
   <213> Homo sapiens
<400> 1
   Met Thr Pro Gly Lys Thr Ser Leu Val Ser Leu Leu Leu Leu Leu Ser
   1               5                   10                  15
   Leu Glu Ala Ile Val Lys Ala Gly Ile Thr Ile Pro Arg Asn Pro Gly
               20                  25                  30
   Cys Pro Asn Ser Glu Asp Lys Asn Phe Pro Arg Thr Val Met Val Asn
           35                  40                  45
   Leu Asn Ile His Asn Arg Asn Thr Asn Thr Asn Pro Lys Arg Ser Ser
       50                  55                  60
   Asp Tyr Tyr Asn Arg Ser Thr Ser Pro Trp Asn Leu His Arg Asn Glu
   65                  70                  75                      80
   Asp Pro Glu Arg Tyr Pro Ser Val Ile Trp Glu Ala Lys Cys Arg His
                   85                  90                  95
   Leu Gly Cys Ile Asn Ala Asp Gly Asn Val Asp Tyr His Met Asn Ser
               100                 105                 110
   Val Pro Ile Gln Gln Glu Ile Leu Val Leu Arg Arg Glu Pro Pro His
           115                 120                 125
   Cys Pro Asn Ser Phe Arg Leu Glu Lys Ile Leu Val Ser Val Gly Cys
       130                 135                 140
   Thr Cys Val Thr Pro Ile Val His His Val Ala
   145                 150                 155
<210> 2
   <211> 155
   <212> PRT
   <213> Homo sapiens
   Xaa57 is Asn or Glu
   Xaa61 is Lys or Arg
   Xaa83 is Glu or Gln
   Xaa91 is Glu or Gln
   Xaa96 is His or Asn
<400> 2
   Met Thr Pro Gly Lys Thr Ser Leu Val Ser Leu Leu Leu Leu Leu Ser
   1               5                   10                  15
   Leu Glu Ala Ile Val Arg Ala Gly Ile Thr Ile Pro Arg Asn Pro Gly
               20                  25                  30
   Cys Pro Asn Ser Glu Asp Lys Asn Phe Pro Arg Thr Val Met Val Asn
           35                  40                  45
   Leu Asn Ile His Asn Arg Asn Thr Xaa Thr Asn Pro Xaa Arg Ser Ser
       50                  55                  60
   Asp Tyr Tyr Asn Arg Ser Thr Ser Pro Trp Asn Leu His Arg Asn Glu
   65                  70                  75                  80
   Asp Pro Xaa Arg Tyr Pro Ser Val Ile Trp Xaa Ala Lys Cys Arg Xaa
                   85                  90                  95
   Leu Gly Cys Ile Asn Ala Asp Gly Asn Val Asp Tyr His Met Asn Ser
               100                 105                 110
   Val Pro Ile Gln Gln Glu Ile Leu Val Leu Arg Arg Glu Pro Pro His
           115                 120                 125
   Cys Pro Asn Ser Phe Arg Leu Glu Lys Ile Leu Val Ser Val Gly Cys
       130                 135                 140
   Thr Cys Val Thr Pro Ile Val His His Val Gln
   145                 150                 155
<210> 3
   <211> 136
   <212> PRT
   <213> Artificial
   Xaa38 is Asn or Glu
   Xaa42 is Lys or Arg
   Xaa64 is Glu or Gln
   Xaa72 is Glu or Gln
   Xaa77 is His or Asn
<400> 3
   Ile Val Arg Ala Gly Ile Thr Ile Pro Arg Asn Pro Gly Cys Pro Asn
   1               5                   10                  15
   Ser Glu Asp Lys Asn Phe Pro Arg Thr Val Met Val Asn Leu Asn Ile
               20                  25                  30
   His Asn Arg Asn Thr Xaa Thr Asn Pro Xaa Arg Ser Ser Asp Tyr Tyr
           35                  40                  45
   Asn Arg Ser Thr Ser Pro Trp Asn Leu His Arg Asn Glu Asp Pro Xaa
       50                  55                  60
   Arg Tyr Pro Ser Val Ile Trp Xaa Ala Lys Cys Arg Xaa Leu Gly Cys
   65                  70                  75                  80
   Ile Asn Ala Asp Gly Asn Val Asp Tyr His Met Asn Ser Val Pro Ile
                   85                  90                  95
   Gln Gln Glu Ile Leu Val Leu Arg Arg Glu Pro Pro His Cys Pro Asn
               100                 105                 110
   Ser Phe Arg Leu Glu Lys Ile Leu Val Ser Val Gly Cys Thr Cys Val
           115                 120                 125
   Thr Pro Ile Val His His Val Glu
       130                 135
<210> 4
   <211> 133
   <212> PRT
   <213> Homo sapiens
<400>